# EUROPEAN PATENT SPECIFICATION

(11) **EP 1 235 594 B1**
(45) Date of publication and mention of the grant of the patent: **13.06.2012**
(21) Application number: 00962721.7
(22) Date of filing: 25.09.2000
(51) Int. Cl.: A61K 47/48

(54) **INHIBITION OF SECRETION FROM NON-NEURONAL CELLS**
INHIBIERUNG DER SEKRETION VON NICHT-NEURONALEN ZELLEN
INHIBITION DE LA SECRETION DE CELLULES NON NEURONALES

(30) Priority: 23.09.1999 GB 9922554
(43) Date of publication of application: 04.09.2002
(60) Divisional application: 09157032.5
(73) Proprietor: Syntaxin Limited, Abingdon Oxfordshire OX14 3YS (GB)
(72) Inventor: FOSTER, Keith Alan, Microbiological Res. Authority, Salisbury SP4 0JG (GB); CHADDOCK, John A., Microbiological Res. Authority, Salisbury SP4 0JG (GB); PURKISS, John R., Immunopharmacology Group, Southampton SO16 6YD (GB); QUINN, Conrad, Padraig,, Atlanta, GA 30333 (US)
(74) Representative: MacLean, Martin Robert
(86) International application number: PCT/GB2000/003669
(87) International publication number: WO 2001/021213

(56) References cited:
- WO-A-00/10598
- WO-A-00/61192
- WO-A-01/14570
- WO-A-01/18038
- WO-A-94/21300
- WO-A-96/33273
- WO-A-98/07864
- WO-A1-95/33850
- US-A1- 2001 018 049
- CHADDOCK J A ET AL: "INHIBITION OF VESICULAR SECRETION IN BOTH NEURONAL AND NONNEURONAL CELLS BY A RETARGETED ENDOPEPTIDASE DERIVATIVE OF CLOSTRIDIUM BOTULINUM NEUROTOXIN TYPE A" INFECTION AND IMMUNITY,AMERICAN SOCIETY FOR MICROBIOLOGY. WASHINGTON,US, vol. 68, no. 5, May 2000 (2000-05), pages 2587-2593, XP001010268 ISSN: 0019-9567
- NEMOZ-GAILLARD E ET AL: "EXPRESSION OF SNARE PROTEINS IN ENTEROENDOCRINE CELL LINES AND FUNCTIONAL ROLE OF TETANUS TOXIN-SENSITIVE PROTEINS IN CHOLECYSTOKININ RELEASE" FEBS LETTERS,ELSEVIER SCIENCE PUBLISHERS, AMSTERDAM,NL, vol. 425, no. 1, 20 March 1998 (1998-03-20), pages 66-70, XP001012518 ISSN: 0014-5793
- BIZZINI, BERNARD: "Investigation of the mode of action of tetanus toxin with the aid of hybrid molecules consisting in part of tetanus toxin -derived fragments" FEMS SYMP. (1984), 24(BACT. PROTEIN TOXINS), 427-34, XP001014243
- KURAZONO H ET AL: "MINIMAL ESSENTIAL DOMAINS SPECIFYING TOXICITY OF THE LIGHT CHAINS OF TETANUS TOXIN AND BOTULINUM NEUROTOXIN TYPE A", JOURNAL OF BIOLOGICAL CHEMISTRY, THE AMERICAN SOCIETY OF BIOLOGICAL CHEMISTS, INC, US, vol. 267, no. 21, 25 July 1992 (1992-07-25), pages 14721-14729, XP002047910, ISSN: 0021-9258
- SHONE C C ET AL: "A 50-K DA FRAGMENT FROM THE NH2-TERMINUS OF THE HEAVY SUBUNIT OF CLOSTRIDIUM BOTULINUM TYPE A NEUROTOXIN FORMS CHANNELS IN LIPID VESICLES", EUROPEAN JOURNAL OF BIOCHEMISTRY, BLACKWELL PUBLISHING, BERLIN, DE, vol. 167, 1 January 1987 (1987-01-01), pages 175-180, XP000986496, ISSN: 0014-2956, DOI: 10.1111/J.1432-1033.1987.TB13320.X
- BLAUSTEIN R O ET AL: "THE N-TERMINAL HALF OF THE HEAVY CHAIN OF BOTULINUM TYPE A NEUROTOXIN FORMS CHANNELS IN PLANAR PHOSPHOLIPID BILAYERS", FEBS LETTERS, ELSEVIER, AMSTERDAM, NL, vol. 226, no. 1, 1 December 1987 (1987-12-01), pages 115-120, XP000942368, ISSN: 0014-5793, DOI: 10.1016/0014-5793(87)80562-8

## Description

The present invention relates to uses for treatment of disease by inhibition of secretion from non-neuronal inflammatory cells, and to agents therefor. The present invention relates to treatment of diseases dependent upon the exocytotic activity of inflammatory cells.

Exocytosis is the fusion of secretory vesicles with the plasma membrane and results in the discharge of vesicle content - a process also known as cell secretion. Exocytosis can be constitutive or regulated. Constitutive exocytosis is thought to occur in every cell type whereas regulated exocytosis occurs from specialised cells.

The understanding of the mechanisms involved in exocytosis has increased rapidly, following the proposal of the SNARE hypothesis (Rothman, 1994, Nature 372, 55-63). This hypothesis describes protein markers on vesicles, which recognise target membrane markers. These so-called cognate SNARES (denoted v-SNARE for vesicle and t-SNARE for target) facilitate docking and fusion of vesicles with the correct membranes, thus directing discharge of the vesicular contents into the appropriate compartment. Key to the understanding of this process has been the identification of the proteins involved. Three SNARE protein families have been identified for exocytosis: SNAP-25 and SNAP-23, and syntaxins are the t-SNARE families in the membrane; and VAMPs (vesicle-associated membrane protein), including synaptobrevin and cellubrevin, are the v-SNARE family on secretory vesicles. Key components of the fusion machinery including SNARES are involved in both regulated and constitutive exocytosis (De Camilli, 1993, Nature, 364, 387-388).

The clostridial neurotoxins are proteins with molecular masses of the order of 150kDa. They are produced by various species of the genus *Clostridium,* most importantly *C*. *tetani* and several strains of *C*. *botulinum.* There are at present eight different classes of the neurotoxins known: tetanus toxin and botulinum neurotoxin in its serotypes A, B, C₁, D, E, F and G, and they all share similar structures and modes of action. The clostridial neurotoxins are synthesized by the bacterium as a single polypeptide that is modified post-translationally to form two polypeptide chains joined together by a disulphide bond. The two chains are termed the heavy chain (H) which has a molecular mass of approximately 100 kDa and the light chain (LC) which has a molecular mass of approximately 50 kDa. The clostridial neurotoxins are highly selective for neuronal cells, and bind with high affinity thereto [see Black, J.D. and Dolly, J.O. (1987) Selective location of acceptors for BoNT/A in the central and peripheral nervous systems. Neuroscience, 23, pp.767-779*;* Habermann, E. and Dreyer, F. (1986) Clostridial neurotoxins:handling and action at the cellular and molecular level. Curr. Top. Microbiol. Immunol. 129, pp. 93-179*;* and Sugiyama, H. (1980) Clostridiumbotulinumneurotoxin. Microbiol. Rev., 44, pp.419-448 (and internally cited references)].

The functional requirements of neurointoxication by the clostridial neurotoxins can be assigned to specific domains within the neurotoxin structure. The clostridial neurotoxins bind to an acceptor site on the cell membrane of the motor neuron at the neuromuscular junction and, following binding to the highly specific receptor, are internalised by an endocytotic mechanism. The specific neuromuscular junction binding activity of clostridial neurotoxins is known to reside in the carboxy-terminal portion of the heavy chain component of the dichain neurotoxin molecule, a region known as H_{C}. The internalised clostridial neurotoxins possess a highly specific zinc-dependent endopeptidase activity that hydrolyses a specific peptide bond in at least one of three protein families, synaptobrevin, syntaxin or SNAP-25, which are crucial components of the neurosecretory machinery. The zinc-dependent endopeptidase activity of clostridial neurotoxins is found to reside in the L-chain (LC). The amino-terminal portion of the heavy chain component of the dichain neurotoxin molecule, a region known as H_{N}, is responsible for translocation of the neurotoxin, or a portion of it containing the endopeptidase activity, across the endosomal membrane following internalisation, thus allowing access of the endopeptidase to the neuronal cytosol and its substrate protein(s). The result of neurointoxication is inhibition of neurotransmitter release from the target neuron due to prevention of release of synaptic vesicle contents.

The mechanism by which the H_{N} domain effects translocation of the endopeptidase into the neuronal cytosol is not fully characterised but is believed to involve a conformational change, insertion into the endosomal membrane and formation of some form of channel or pore through which the endopeptidase can gain access to the neuronal cytosol. Following binding to its specific receptor at the neuronal surface pharmacological and morphologic evidence indicate that the clostridial neurotoxins enter the cell by endocytosis [Black & Dolly (1986) J. Cell Biol. 103, 535-44] and then have to pass through a low pH step for neuron intoxication to occur [Simpson et al (1994) J. Pharmacol Exp. Ther., 269, 256-62]. Acidic pH does not activate the toxin directly via a structural change, but is believed to trigger the process of LC membrane translocation from the neuronal endosomal vesicle lumen to the neuronal cytosol [Montecucco et al (1994) FEBS Lett. 346, 92-98]. There is a general consensus that toxin-determined channels are related to the translocation process into the cytosol [Schiavo & Montecucco (1997) in Bacterial Toxins (ed. K. Aktories)]. This model requires that the H_{N} domain forms a transmembrane hydrophobic pore across the acidic vesicle membrane that allows the partially unfolded LC passage through to the cytosol. The requisite conformational change is believed to be triggered by environmental factors in the neuronal endosomal compartment into which the neurotoxin is internalised, and a necessary feature of the binding domain of the H_{C} is to target binding sites which enable internalisation into the appropriate endosomal compartment. Therefore clostridial neurotoxins have evolved to target cell surface moieties that fulfil this requirement.

Hormones are chemical messengers that are secreted by the endocrine glands of the body. They exercise specific physiological actions on other organs to which they are carried by the blood. The range of processes regulated by hormones includes various aspects of homeostasis (e.g. insulin regulates the concentration of glucose in the blood), growth (e.g. growth hormone promotes growth and regulates fat, carbohydrate and protein metabolism), and maturation (e.g. sex hormones promote sexual maturation and reproduction). Endocrine hyperfunction results in disease conditions which are caused by excessive amounts of a hormone or hormones in the bloodstream. The causes of hyperfunction are classified as neoplastic, autoimmune, iatrogenic and inflammatory. The endocrine hyperfunction disorders are a complex group of diseases, not only because there is a large number of glands that can cause a pathology (e.g. anterior pituitary, posterior pituitary, thyroid, parathyroid, adrenal cortex, adrenal medulla, pancreas, ovaries, testis) but because many of the glands produce more than one hormone (e.g. the anterior pituitary produces corticotrophin, prolactin, luteinizing hormone, follicle stimulating hormone, thyroid stimulating hormone and gonadotrophins). The majority of disorders that cause hormone excess are due to neoplastic growth of hormone producing cells. However, certain tumours of non-endocrine origin can synthesise hormones causing endocrine hyperfunction disease symptoms. The hormone production under these conditions is termed "ectopic". Surgical removal or radiation induced destruction of part or all of the hypersecreting tissue is frequently the treatment of choice. However, these approaches are not always applicable, result in complete loss of hormone production or have to be repeated due to re-growth of the secreting tissue.

A further level of complexity in endocrine hyperfunction disorders arises in a group of conditions termed multiple endocrine neoplasia (MEN) where two or more endocrine glands are involved. The multiple endocrine neoplasia syndromes (MEN1 and MEN2) are familial conditions with an autosomal dominant pattern of inheritance. MEN1 is characterised by the association of parathyroid hyperplasia, pancreatic endocrine tumours, and pituitary adenomas, and has a prevalence of about 1 in 10000. MEN2 is the association of medullary cell carcinoma of the thyroid and phaeochromocytoma, though parathyroid hyperplasia may also occur in some sufferers.

Most of the morbidity associated with MEN1 is due to the effects of pancreatic endocrine tumours. Often surgery is not possible and the therapeutic aim is to reduce hormone excess. Aside from reducing tumour bulk, which is often precluded, inhibition of hormone secretion is the preferred course of action. Current procedures include subcutaneous application of the somatostatin analogue, octreotide. However, this approach is only temporarily effective, and the success diminishes over a period of months.

Many further disease states are known that involve secretion from other non-endocrine, non-neuronal cells. It would accordingly be desirable to treat, reduce or prevent secretion by non-neuronal cells, such as hyperfunction of the endocrine cells that causes or leads to these disease conditions.

The activity of the botulinum neurotoxins is exclusively restricted to inhibition of neurotransmitter release from neurons. This is due to the exclusive expression of high affinity binding sites for clostridial neurotoxins on neuronal cells [see Daniels-Holgate, P.U. and Dolly, J.O. (1996) Productive and non-productive binding of botulinum neurotoxin to motor nerve endings are distinguished by its heavy chain. J. Neurosci. Res. 44, 263-271].

Non-neuronal cells do not possess the high affinity binding sites for clostridial neurotoxins, and are therefore refractory to the inhibitory effects of exogenously applied neurotoxin. Simple application of clostridial neurotoxins to the surface of non-neuronal cells does not therefore lead to inhibition of secretory vesicle exocytosis.

The productive binding or lack of productive binding of clostridial neurotoxins thereby defines neuronal and non-neuronal cells respectively.

In addition to lacking high affinity binding sites for clostridial neurotoxins, absence of the correct internalisation and intracellular routing mechanism, or additional factors that are not yet understood, would prevent clostridial neurotoxin action in non-neuronal cells.

It is known from WO96/33273 that hybrid clostridial neurotoxins endopeptidases can be prepared and that these hybrids effectively inhibit release of neurotransmitters from neuronal cells to which they are targeted, such as pain transmitting neurons. WO96/33273 describes the activity of hybrids only in neuronal systems where neuronal mechanisms of internalisation and vesicular routing are operational.

Non-neuronal cells are, however, refractory to the effects of clostridial neurotoxins, since simple application of clostridial neurotoxins to the surface of non-neuronal cells does not lead to inhibition of secretory vesicle exocytosis. This insensitivity of non-neuronal cells to clostridial neurotoxins may be due to absence of the requisite receptor, absence of the correct internalisation & intracellular routing mechanism, or additional factors that are not yet understood.

WO95/17904 describes the use of *C*. *botulinum* holotoxin in the treatment of various disorders such as excessive sweating, lacrimation and mucus secretion, and pain. WO95/17904 describes treatment by targeting neuronal cells

It is an object of the present invention to provide uses and agents for inhibition of secretion from non-neuronal inflammatory cells.

WO00/10598, published in the priority interval, describes treating mucus hypersecretion. Specific disease states caused by or exacerbated by mucus hypersecretion are localised to the airways, and are treatable by topical administration to the airways or to a selected region or to a selected portion of the airways. The present invention excludes that previous invention.

Accordingly, the present invention is based upon the use of an agent that inhibits the exocytotic machinery in neuronal cells and which surprisingly has been found to be effective at inhibiting exocytotic processes in non-neuronal inflammatory cells.

A first aspect of the invention thus provides the use of an agent for the manufacture of a medicament for inhibiting secretion from a non-neuronal inflammatory cell, said agent comprising at least first and second domains, wherein the first domain cleaves one or more proteins essential to exocytosis and the second domain translocates the first domain into the inflammatory cell.

Advantageously, the invention provides for inhibition of secretion from non-neuronal inflammatory cells, and enables treatment of disease caused, exacerbated or maintained by such secretion.

An agent for use in the invention is suitably prepared by replacement of the cell-binding H_{C} domain of a clostridial neurotoxin with a ligand capable of binding to the surface of non-neuronal cells. Surprisingly, this agent is capable of inhibiting the exocytosis of a variety of secreted substances from non-neuronal inflammatory cells. By covalently linking a clostridial neurotoxin, or a hybrid of two clostridial neurotoxins, in which the H_{C} region of the H-chain has been removed or modified, to a new molecule or moiety, the Targeting Moiety (TM), an agent is produced that binds to a binding site (BS) on the surface of the relevant non-neuronal secretory cells. A further surprising aspect of the present invention is that if the L-chain of a clostridial neurotoxin, or a fragment, variant or derivative of the L-chain containing the endopeptidase activity, is covalently linked to a TM which can also effect internalisation of the L-chain, or a fragment of the endopeptidase activity, into the cytoplasm of a non-neuronan inflammatory secretory cell, this also produces an agent capable of inhibiting secretion. Thus, the present invention overcomes the insusceptibility of non-neuronal inflammatory cells to the inhibitory effects of clostridial neurotoxins.

An example of an agent for use in the invention is a polypeptide comprising first and second domains, wherein said first domain cleaves one or more vesicle or plasma-membrane associated proteins essential to neuronal exocytosis and wherein said second domain translocates the polypeptide into the cell or translocates at least that portion responsible for the inhibition of exocytosis into the non-neuronal inflammatory cell. The polypeptide can be derived from a neurotoxin in which case the polypeptide is typically free of clostridial neurotoxin and free of any clostridial neurotoxin precursor that can be converted into toxin by proteolytic action, being accordingly substantially non-toxic and suitable for therapeutic use. Accordingly, the invention may thus use polypeptides containing a domain equivalent to a clostridial toxin light chain and a domain providing the translocation function of the H_{N} of a clostridial toxin heavy chain, whilst lacking the functional aspects of a clostridial toxin H_{C} domain.

In use of the invention, the polypeptide is for administration *in vivo* to a patient, wherein the first domain is translocated into a non-neuronal inflammatory cell by action of the second domain and cleaves one or more vesicle or plasma-membrane associated proteins essential to the specific cellular process of exocytosis, and cleavage of these proteins results in inhibition of exocytosis, thereby resulting in inhibition of secretion, typically in a non-cytotoxic manner.

The polypeptide of the invention may be obtained by expression of a recombinant nucleic acid, preferably a DNA, and can be a single polypeptide, that is to say not cleaved into separate light and heavy chain domains or two polypeptides linked for example by a disulphide bond.

The first domain preferably comprises a clostridial toxin light chain or a functional fragment or variant of a clostridial toxin light chain. The fragment is optionally an N-terminal, or C-terminal fragment of the light chain, or is an internal fragment, so long as it substantially retains the ability to cleave the vesicle or plasma-membrane associated protein essential to exocytosis. The minimal domains necessary for the activity of the light chain of clostridial toxins are described in J. Biol. Chem., Vol.267, No. 21, July 1992, pages 14721-14729. The variant has a different peptide sequence from the light chain or from the fragment, though it too is capable of cleaving the vesicle or plasma-membrane associated protein. It is conveniently obtained by insertion, deletion and/or substitution of a light chain or fragment thereof. A variety of variants are possible, including (i) an N-terminal extension to a clostridial toxin light chain or fragment (ii) a clostridial toxin light chain or fragment modified by alteration of at least one amino acid (iii) a C-terminal extension to a clostridial toxin light chain or fragment, or (iv) combinations of 2 or more of (i)-(iii). In further embodiments of the invention, the variant contains an amino acid sequence modified so that (a) there is no protease sensitive region between the LC and H_{N} components of the polypeptide, or (b) the protease sensitive region is specific for a particular protease. This latter embodiment is of use if it is desired to activate the endopeptidase activity of the light chain in a particular environment or cell, though, in general, the polypeptides of the invention are in an active form prior to administration.

The first domain preferably exhibits endopeptidase activity specific for a substrate selected from one or more of SNAP-25, synaptobrevin/VAMP and syntaxin. The clostridial toxin from which this domain can be obtained or derived is preferably botulinum toxin or tetanus toxin. The polypeptide can further comprise a light chain or fragment or variant of one toxin type and a heavy chain or fragment or variant of another toxin type.

The second domain preferably comprises a clostridial toxin heavy chain H_{N} portion or a fragment or variant of a clostridial toxin heavy chain H_{N} portion. The fragment is optionally an N-terminal or C-terminal or internal fragment, so long as it retains the function of the H_{N} domain. Teachings of regions within the H_{N} responsible for its function are provided for example in Biochemistry 1995, 34, pages 15175-15181 and Eur. J. Biochem, 1989, 185, pages 197-203. The variant has a different sequence from the H_{N} domain or fragment, though it too retains the function of the H_{N} domain. It is conveniently obtained by insertion, deletion and/or substitution of a H_{N} domain or fragment thereof, and examples of variants include (i) an N-terminal extension to a H_{N} domain or fragment, (ii) a C-terminal extension to a H_{N} domain or fragment, (iii) a modification to a H_{N} domain or fragment by alteration of at least one amino acid, or (iv) combinations of 2 or more of (i)-(iii). The clostridial toxin is preferably botulinum toxin or tetanus toxin.

In preparation of the polypeptides by recombinant means, methods employing fusion proteins can be employed, for example a fusion protein comprising a fusion of (a) a polypeptide of the invention as described above with (b) a second polypeptide adapted for binding to a chromatography matrix so as to enable purification of the fusion protein using said chromatography matrix. It is convenient for the second polypeptide to be adapted to bind to an affinity matrix, such as glutathione Sepharose, enabling rapid separation and purification of the fusion protein from an impure source, such as a cell extract or supernatant.

One second purification polypeptide is glutathione-S-transferase (GST), and others may be chosen so as to enable purification on a chromatography column according to conventional techniques.

In a second aspect of the invention there is provided a use of an agent of the invention inhibiting secretion from selected non-neuronal inflammatory cells responsible for regulated secretion.

In a third aspect of the invention there is provided for the manufacture of a medicament for treatment of disease resulting, or caused or maintained by secretions from non-neuronal inflammatory cells

In further aspects of the invention there are provided agents of the invention targeted to non-neuronal inflammatory cells responsible for secretion.

An agent of the invention is used for the manufacture of a medicament for treatment of conditions resulting from secretions of inflammatory cells, for example allergies. Ligands suitable to target agent to these cells include (i) for mast cells, complement receptors in general, including C4 domain of the Fc IgE, and antibodies/ligands to the C3a/C4a-R complement receptor; (ii) for eosinophils, antibodies/ligands to the C3a/C4a-R complement receptor, anti VLA-4 monoclonal antibody, anti-IL5 receptor, antigens or antibodies reactive toward CR4 complement receptor; (iii) for macrophages and monocytes, macrophage stimulating factor, (iv) for macrophages, monocytes and neutrophils, bacterial LPS and yeast B-glucans which bind to CR3, (v) for neutrophils, antibody to OX42, an antigen associated with the iC3b complement receptor, or IL8; (vi) for fibroblasts, mannose 6-phosphate/insulin-like growth fartor-beta (M6P/IGF-II) receptor and PA2.26, antibody to a cell-surface receptor for active fibroblasts in mice. Diseases thus treatable according to the invention include diseases selected from allergies (seasonal allergic rhinitis (hay fever), allergic conjunctivitis, vasomotor rhinitis and food allergy), eosinophilia, asthma, rheumatoid arthritis, systemic lupus erythematosus, discoid lupus erythematosus, ulcerative colitis, Crohn's disease, haemorrhoids, pruritus, glomerulonephritis, hepatitis, pancreatitis, gastritis, vasculitis, myocarditis, psoriasis, eczema, chronic radiation-induced fibrosis, lung scarring and other fibrotic disorders.

VAMP expression has been demonstrated in B-lymphocytes [see Olken, S. K.and Corley, R. B. 1998, Mol. Biol. Cell. 9, 207a]. Thus, an agent according to the present invention, when targeted to a B-lymphocyte and following internalisation and retrograde transport, may exert its inhibitory effect on such target cells.

In use of the invention, a Targeting moiety (TM) provides specificity for the BS on the relevant non-neuronal inflammatory secretory cells. The TM component of the agent can comprise one of many cell binding molecules, including, but not limited to, antibodies, monoclonal antibodies, antibody fragments (Fab, F(ab)'₂, Fv, ScFv, etc.), lectins, hormones, cytokines, growth factors, peptides, carbohydrates, lipids, glycons, nucleic acids or complement components.

The TM is selected in accordance with the desired non-neuronal-inflammatory cell-type to which the agent of the present invention is to be targeted, and preferably has a high specificity and/or affinity for non-neuronal inflammatory target cells. Preferably, the TM does not substantially bind to neuronal cells of the presynaptic muscular junction, and thus the agent is substantially non-toxic in that it is not capable of effecting muscular paralysis. This is in contrast to clostridial holotoxin which targets the presynaptic muscular junction and effects muscular paralysis. In addition, preferably the TM does not substantially bind to neuronal peripheral sensory cells, and thus the agent does not exert any substantial analgesic effect. Preferably, the TM does not substantially bind to neuronal cells, and does not therefore permit the agent to exert an inhibitory effect on secretion in a neuronal cell.

It is known in the art that the H_{C} portion of the neurotoxin molecule can be removed from the other portion of the H-chain, known as H_{N}, such that the H_{N} fragment remains disulphide linked to the L-chain of the neurotoxin providing a fragment known as LH_{N}. Thus, in one embodiment of the present invention the LH_{N} fragment of a clostridial neurotoxin is covalently linked, using linkages which may include one or more spacer regions, to a TM.

In another embodiment of the invention, the H_{C} domain of a clostridial neurotoxin is mutated, blocked or modified, e.g. by chemical modification, to reduce or preferably incapacitate its ability to bind the neurotoxin to receptors at the neuromuscular junction. This modified clostridial neurotoxin is then covalently linked, using linkages which may include one or more spacer regions, to a TM.

In another embodiment of the invention, the heavy chain of a clostridial neurotoxin, in which the H_{C} domain is mutated, blocked or modified, e.g. by chemical modification, to reduce or preferably incapacitate its ability to bind the neurotoxin to receptors at the neuromuscular junction, is combined with the L-chain of a different clostridial neurotoxin. This hybrid, modified clostridial neurotoxin is then covalently linked, using linkages which may include one or more spacer regions, to a TM.

In another embodiment of the invention, the H_{N} domain of a clostridial neurotoxin is combined with the L-chain of a different clostridial neurotoxin. This hybrid LH_{N} is then covalently linked, using linkages which may include one or more spacer regions, to a TM.

In another embodiment of the invention, the light chain of a clostridial neurotoxin, or a fragment of the light chain containing the endopeptidase activity, is covalently linked, using linkages which may include one or more spacer regions, to a TM which can also effect the internalisation of the L-chain, or a fragment of the L-chain containing the endopeptidase activity, into the cytoplasm of the relevant non-neuronal cells responsible for secretion.

In another embodiment of the invention, the light chain of a clostridial neurotoxin, or a fragment of the light chain containing the endopeptidase activity, is covalently linked, using linkages which may include one or more spacer regions, to a translocation domain to effect transport of the endopeptidase fragment into the cytosol. Examples of translocation domains derived from bacterial neurotoxins are as follows:

| | |
|---|---|
| Botulinum type A neurotoxin | - amino acid residues (449-871) |
| Botulinum type B neurotoxin | - amino acid residues (441-858) |
| Botulinum type C neurotoxin | - amino acid residues (442-866) |
| Botulinum type D neurotoxin | - amino acid residues (446-862) |
| Botulinum type E neurotoxin | - amino acid residues (423-845) |
| Botulinum type F neurotoxin | - amino acid residues (440-864) |
| Botulinum type G neurotoxin | - amino acid residues (442-863) |
| Tetanus neurotoxin | - amino acid residues (458-879) |

other clostridial sources include - *C*. *butyricum,* and *C. argentinense* [for the genetic basis of toxin production in *Clostridium botulinum* and *C*. *tetani,* see Henderson et al (1997) in The Clostridia: Molecular Biology and Pathogenesis, Academic press*].*

In addition to the above translocation domains derived from clostridial sources, other non-clostridial sources may be employed in an agent according to the present invention. These include, for example, diphtheria toxin [London, E. (1992) Biochem. Biophys. Acta., 1112, pp.25-51], Pseudomonas exotoxin A [Prior et al (1992) Biochem., 3 1, pp. 3555-3559]*,* influenza virus haemagglutinin fusogenic peptides [Wagner et al (1992) PNAS, 89, pp. 7934-79381*,* and amphiphilic peptides [Murata et al (1992) Biochem., 3 1, pp. 1986-1992]*.*

In use, the domains of an agent according to the present invention are associated with each other. In one embodiment, two or more of the Domains may be joined together either directly (eg. by a covalent linkage), or via a linker molecule. Conjugation techniques suitable for use in the present invention have been well documented:-
Chemistry of protein conjugation and cross-linking. Edited by Wong, S. S. 1993, CRC Press Inc., Florida; and
Bioconjugate techniques, Edited by Hermanson, G. T. 1996, Academic Press, London, UK.

Direct linkage of two or more of Domains is now described with reference to clostridial neurotoxins and to the present Applicant's nomenclature of clostridial neurotoxin domains, namely Domain B (contains the binding domain), Domain T (contains the translocation domain) and Domain E (contains the protease domain), although no limitation thereto is intended.

In one embodiment of the present invention, Domains E and T may be mixed together in equimolar quantities under reducing conditions and covalently coupled by repeated dialysis (eg. at 4° C, with agitation), into physiological salt solution in the absence of reducing agents. At this stage, in contrast to Example 6 of WO94/21300, the E-T complex is not blocked by iodoacetamide, therefore any remaining free -SH groups are retained.

Domain B is then modified, for example, by derivatisation with SPDP followed by subsequent reduction. In this reaction, SPDP does not remain attached as a spacer molecule to Domain B, but simply increases the efficiency of this reduction reaction.

Reduced domain B and the E-T complex may then be mixed under nonreducing conditions (eg. at 4 °C) to form a disulphide-linked E-T-B "agent". In another embodiment, a coupled E-T complex may be prepared according to Example 6 of WO94/21300, including the addition of iodoacetamide to block free sulphydryl groups. However, the E-T complex is not further derivatised, and the remaining chemistry makes use of the free amino (-NH₂) groups on amino acid side chains (eg. lysine, and arginine amino acids).

Domain B may be derivatised using carbodiimide chemistry (eg. using EDC) to activate carboxyl groups on amino acid side chains (eg. glutamate, and aspartate amino acids), and the E-T complex mixed with the derivatised Domain B to result in a covalently coupled (amide bond) E-T-B complex.

Suitable methodology for the creation of such an agent is, for example, as follows:-

Domain B was dialysed into MES buffer (0.1 M MES, 0.1 M sodium chloride, pH 5.0) to a final concentration of 0.5mg/ml. EDAC (1-ethyl-3-[3-dimethylaminopropyl] carbodiimide hydrochloride) was added to final concentrations of 0.2 mg/ml and reacted for 30 min at room temperature. Excess EDAC was removed by desalting over a MES buffer equilibrated PD-10 column (Pharmacia). The derivatised domain B was concentrated (to > 2mg/ml) using Millipore Biomax 10 concentrators. The E-T complex (1 mg/ml) was mixed for 16 hours at 4 °C, and the E-T-B complex purified by size-exclusion chromatography over a Superose 12 HR10/30 column (Pharmacia) to remove unreacted Domain B (column buffer: 50mM sodium phosphate pH6.5 + 20mM NaCl).

As an alternative to direct covalent linkage of the various Domains of an agent according to the present invention, suitable spacer molecules may be employed. The term linker molecule is used synonymously with spacer molecule. Spacer technology was readily available prior to the present application.

For example, one particular coupling agent (SPDP) is described in Example 6 of WO94/21300 (see lines 3-5 on page 16). In Example 6, SPDP is linked to an E-T complex, thereby providing an E-T complex including a linker molecule. This complex is then reacted a Domain B, which becomes attached to the E-T complex via the linker molecule. In this method, SPDP results in a spacing region of approximately 6.8 Angstroms between different Domains of the "agent" of the present invention.

A variant of SPDP known as LC-SPDP is identical in all respects to SPDP but for an increased chain length. LC-SPDP may be used to covalently link two Domains of the "agent" of the present invention resulting in a 15.6 Angstrom spacing between these Domains.

Examples of spacer molecules include, but are not limited to:-
- (GGGGS)₂, elbow regions of Fab: - [see Anand et al. (1991) J. Biol. Chem. 266, 21874-9];
- (GGGGS)₃: - [see Brinkmann et al. (1991) Proc. Natl. Acad. Sci. 88, 8616-2O];
- the interdomain linker of cellulase: - [see Takkinen et al. (1991) Protein Eng, 4, 837-841];
- PPPIEGR: - [see Kim (1993) Protein Science, 2, 348-356];
- Collagen-like spacer: - [see Rock (1992) Protein Engineering, vol 5, No 6, pp583-591]; and
- Trypsin-sensitive diphtheria toxin peptide: - [see O'Hare (1990) FEBS, vol 273, No 1,2, pp 200-204].

In a further embodiment of the present invention, an agent having the structure E-X-T-X-B, where "X" is a spacer molecule between each domain, may be prepared, for example, as follows:-

Domain E is derivatised with SPDP, but not subsequently reduced. This results in an SPDP-derivatised Domain E.

Domain T is similarly prepared, but subsequently reduced with 10mM dithiothreitol (DTT). The 10mM DTT present in the Domain T preparation, following elution from the QAE column (see Example 6 in WO94/21300), is removed by passage of Domain T through a sephadex G-25 column equilibrated in PBS.

Domain T free of reducing agent is then mixed with the SPDP-derivatised Domain E, with agitation at 4 °C for 16 hours. E-T complex is isolated from free Domain E and from free Domain T by size-exclusion chromatography (Sephadex G-150). Whereafter, the same procedure can be followed as described in Example 6 of WO94/21300 for rederivatisation of the E-T complex with SPDP, and subsequent coupling thereof to the free sulphydryl on Domain B.

The agents according to the present invention may be prepared recombinantly.

In one embodiment, the preparation of a recombinant agent may involve arrangement of the coding sequences of the selected TM and clostridial neurotoxin component in a single genetic construct. These coding sequences may be arranged in-frame so that subsequent transcription and translation is continuous through both coding sequences and results in a fusion protein. All constructs would have a 5' ATG codon to encode an N-terminal methionine, and a C-terminal translational stop codon.

Thus, a the light chain of a clostridial neurotoxin (or a fragment of the light chain containing the endopeptidase activity) may be expressed recombinantly as a fusion protein with a TM which can also effect the internalisation of the L-chain (or a fragment thereof) into the cytoplasm of the relevant non-neuronal cells responsible for secretion. The expressed fusion protein may also include one or more spacer regions.

In the case of an agent based on clostridial neurotoxin, the following information would be required to produce said agent recombinantly:-
(i) DNA sequence data relating to a selected TM;
(ii) DNA sequence data relating to the clostridial neurotoxin component; and
(iii) a protocol to permit construction and expression of the construct comprising (i) and (ii).

All of the above basic information (i)-(iii) are either readily available, or are readily determinable by conventional methods. For example, both WO98/07864 and WO99/17806 exemplify clostridial neurotoxin recombinant technology suitable for use in the present application.

In addition, methods for the construction and expression of the constructs of the present invention may employ information from the following references and others:-

Lorberboum-Galski, H., FitzGerald, D., Chaudhary, V., Adhya, S., Pastan, I. (1988). Cytotoxic activity of an interleukin 2-Pseudomonas exotoxin chimeric protein produced in Escherichia coli. Proc Natl Acad Sci U S A 85(6):1922-6;

Murphy, J.R. (1988) Diphtheria-related peptide hormone gene fusions: a molecular genetic approach to chimeric toxin development. Cancer Treat Res; 37:123-40;

Williams, D.P., Parker, K., Bacha, P., Bishai, W., Borowski, M., Genbauffe, F., Strom, T.B., Murphy, J.R. (1987). Diphtheria toxin receptor binding domain substitution with interleukin-2: genetic construction and properties of a diphtheria toxin-related interleukin-2 fusion protein. Protein Eng; 1(6):493-8;

Arora, N., Williamson, L.C., Leppla, S.H., Halpern, J.L. (1994). Cytotoxic effects of a chimeric protein consisting of tetanus toxin light chain and anthrax toxin lethal factor in non-neuronal cells J Biol Chem, 269(42):26165-71;

Brinkmann, U., Reiter, Y., Jung, S.H., Lee, B., Pastan, I. (1993). A recombinant immunotoxin containing a disulphide-stabilized Fv fragment. Proc Natl Acad Sci U S A;90(16):7538-42; and

O'Hare, M., Brown, A.N., Hussain, K., Gebhardt, A., Watson, G., Roberts, L.M., Vitetta, E.S., Thorpe, P.E., Lord, J.M. (1990). Cytotoxicity of a recombinant ricin-A-chain fusion protein containing a proteolytically-cleavable spacer sequence. FEBS Lett Oct 29:273(1-2):200-4.

Suitable clostridial neurotoxin sequence information relating to L- and LHN-chains may be obtained from, for example, Kurazono, H. (1992) J. Biol. Chem., vol. 267, No. 21, pp.14721-14729*;* and Popoff, M.R., and Marvaud, J.-C. (1999) The Comprehensive Sourcebook of Bacterial Protein Toxins, 2nd edition (ed. Alouf, J. E., and Freer, J.H.), Academic Press, pp. 174-201*.*

Similarly, suitable TM sequence data are widely available in the art. Alternatively, any necessary sequence data may be obtained by techniques which were well-known to the skilled person.

For example, DNA encoding the TM component may be cloned from a source organism by screening a cDNA library for the correct coding region (for example by using specific oligonucleotides based on the known sequence information to probe the library), isolating the TM DNA, sequencing this DNA for confirmation purposes, and then placing the isolated DNA in an appropriate expression vector for expression in the chosen host.

As an alternative to isolation of the sequence from a library, the available sequence information may be employed to prepare specific primers for use in PCR, whereby the coding sequence is then amplified directly from the source material and, by suitable use of primers, may be cloned directly into an expression vector.

Another alternative method for isolation of the coding sequence is to use the existing sequence information and synthesise a copy, possibly incorporating alterations, using DNA synthesis technology. For example, DNA sequence data may be generated from existing protein and/or RNA sequence information. Using DNA synthesis technology to do this (and the alternative described above) enables the codon bias of the coding sequence to be modified to be optimal for the chosen expression host. This may give rise to superior expression levels of the fusion protein.

Optimisation of the codon bias for the expression host may be applied to the DNA sequences encoding the TM and clostridial components of the construct. Optimisation of the codon bias is possible by application of the protein sequence into freely available DNA/protein database software, eg. programs available from Genetics Computer Group, Inc.

According to a further aspect of the present invention, nucleic acid encoding the light chain of a clostridial neurotoxin (or a fragment of the light chain containing the endopeptidase activity), may be associated with a TM which can also effect the internalisation of the nucleic acid encoding the L-chain (or a fragment thereof) into the cytoplasm of the relevant non-neuronal cells responsible for secretion. The nucleic acid sequence may be coupled to a translocation domain, and optionally to a targeting moiety, by for example direct covalent linkage or via spacer molecule technology. Ideally, the coding sequence will be expressed in the target cell.

Thus, the agent of the present invention may be the expression product of a recombinant gene delivered independently to the preferred site of action of the agent. Gene delivery technologies are widely reported in the literature [reviewed in *"*Advanced Drug Delivery Reviews" Vol. 27, (1997), Elsevier Science Ireland Ltd].

According to another aspect, the present invention therefore provides the use of an effective, non-toxic amount of a compound of the invention for the manufacture of a medicament for treating a condition or disease which is susceptible of treatment with a nucleic acid in a mammal eg. a human. A condition or disease which is susceptible of treatment with a nucleic acid may be for example a condition or disease which may be treated by or requiring gene therapy. The preferred conditions or diseases susceptible to treatment, together with the preferred TMs, have been described previously in this specification. Similarly, the preferred first domains which cleave one or more proteins (eg. SNAP-25, synaptobrevin and syntaxin) essential to exocytosis have been described previously in this specification. The various domains of an agent for use in gene therapy may be directly linked (eg. via a covalent bond) or indirectly linked (eg. via a spacer molecule), as for example previously described in this specification.

The invention further provides a compound of the invention for use as an active therapeutic substance, in particular for use for the manufacture of a medicament for treating a condition or disease as set forth in the present claims.

The invention further provides pharmaceutical compositions comprising an agent of the invention and a pharmaceutically acceptable carrier.

In use the agent will normally be employed in the form of a pharmaceutical composition in association with a human pharmaceutical carrier, diluent and/or excipient, although the exact form of the composition will depend on the mode of administration.

The agent may, for example, be employed in the form of an aerosol or nebulisable solution for inhalation or a sterile solution for parenteral administration, intra-articular administration or intra-cranial administration.

For treating inflammatory cell targets, i.v. injection, sub-cutaneous injection, or surface patch administration are preferred.

In cases of i.v. injection, this should also include the use of pump systems.

The dosage ranges for administration of the compounds of the present invention are those to produce the desired therapeutic effect. It will be appreciated that the dosage range required depends on the precise nature of the conjugate, the route of administration, the nature of the formulation, the age of the patient, the nature, extent or severity of the patient's condition, contraindications, if any, and the judgement of the attending physician.

Suitable daily dosages are in the range 0.0001-1 mg/kg, preferably 0.0001-0.5mg/kg, more preferably 0.002-0.5mg/kg, and particularly preferably 0.004-0.5mg/kg. The unit dosage can vary from less that 1 microgram to 30mg, but typically will be in the region of 0.01 to 1 mg per dose, which may be administered daily or less frequently, such as weekly or six monthly.

Wide variations in the required dosage, however, are to be expected depending on the precise nature of the conjugate, and the differing efficiencies of various routes of administration. For example, oral administration would be expected to require higher dosages than administration by intravenous injection.

Variations in these dosage levels can be adjusted using standard empirical routines for optimization, as is well understood in the art.

Compositions suitable for injection may be in the form of solutions, suspensions or emulsions, or dry powders which are dissolved or suspended in a suitable vehicle prior to use.

Fluid unit dosage forms are typically prepared utilising a pyrogen-free sterile vehicle. The active ingredients, depending on the vehicle and concentration used, can be either dissolved or suspended in the vehicle.

Solutions may be used for all forms of parenteral administration, and are particularly used for intravenous injection. In preparing solutions the compound can be dissolved in the vehicle, the solution being made isotonic if necessary by addition of sodium chloride and sterilised by filtration through a sterile filter using aseptic techniques before filling into suitable sterile vials or ampoules and sealing. Alternatively, if solution stability is adequate, the solution in its sealed containers may be sterilised by autoclaving.

Advantageously additives such as buffering, solubilising, stabilising, preservative or bactericidal, suspending or emulsifying agents and/or local anaesthetic agents may be dissolved in the vehicle.

Dry powders which are dissolved or suspended in a suitable vehicle prior to use may be prepared by filling pre-sterilised drug substance and other ingredients into a sterile container using aseptic technique in a sterile area.

Alternatively the agent and other ingredients may be dissolved in an aqueous vehicle, the solution is sterilized by filtration and distributed into suitable containers using aseptic technique in a sterile area. The product is then freeze dried and the containers are sealed aseptically.

Parenteral suspensions, suitable for intramuscular, subcutaneous or intradermal injection, are prepared in substantially the same manner, except that the sterile compound is suspended in the sterile vehicle, instead of being dissolved and sterilisation cannot be accomplished by filtration. The compound may be isolated in a sterile state or alternatively it may be sterilised after isolation, e.g. by gamma irradiation.

Advantageously, a suspending agent for example polyvinylpyrrolidone is included in the composition to facilitate uniform distribution of the compound.

Compositions suitable for administration via the respiratory tract include aerosols, nebulisable solutions or microfine powders for insufflation. In the latter case, particle size of less than 50 microns, especially less than 10 microns, is preferred. Such compositions may be made up in a conventional manner and employed in conjunction with conventional administration devices.

The agent described in this invention can be used *in vivo,* either directly or as a pharmaceutically acceptable salt, for the manufacture of a medicament for the treatment of conditions involving secretion from non-neuronal inflammatory cells.

The present invention will now be described by reference to the following examples illustrated by the accompanying drawings in which:-
- Fig. 1: shows SDS-PAGE analysis of WGA-LH_{N}/A purification scheme;
- Fig. 2: shows activity of WGA-LH_{N}/A on release of transmitter from HIT-T15 cells;
- Fig. 3: shows correlation of SNAP-25 cleavage with inhibition of neurotransmitter release following application of WGA-LH_{N}/A to HIT-T15 cells;
- Fig. 4: shows activity of WGA-LH_{N}/A on release of [³H]-noradrenaline from undifferentiated PC12 cells;
- Fig. 5: shows a Western blot indicating expression of *rec*LH_{N}/B in *E. coli;*
- Fig. 6: shows *in vitro* cleavage of synthetic VAMP peptide by *rec*LH_{N}/B;
- Fig. 7: shows the effect of low pH and BoNT/B treatment on stimulated von Willebrands Factor (vWF) release from human umbilical vein endothelial cells;
- Fig.8: shows release of [³H]-glucosamine labelled high molecular weight material from LS180 cells; and
- Fig. 9: shows the effect of low pH and BoNT/B treatment on stimulated β-glucuronidase release from differentiated HL60 cells.

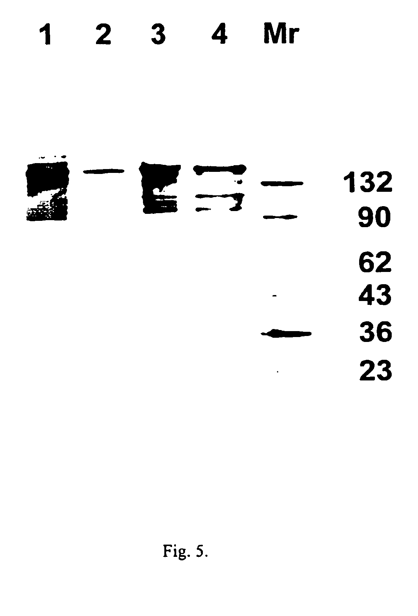

Figures 5-9 are now described in more detail.

Referring to Fig. 5, MBP-LH_{N}/B was expressed in *E. coli* as described in Example 4. Lane 1 represents the profile of the expressed fusion protein in *E. coli.* Lane 2 represents the profile of fusion protein expression in the crude *E. coli* lysate. Lane 3 represents the profile of the MBP-LH_{N}/B following purification by immobilised amylose. Molecular weights in kDa are indicated to the right side of the Figure.

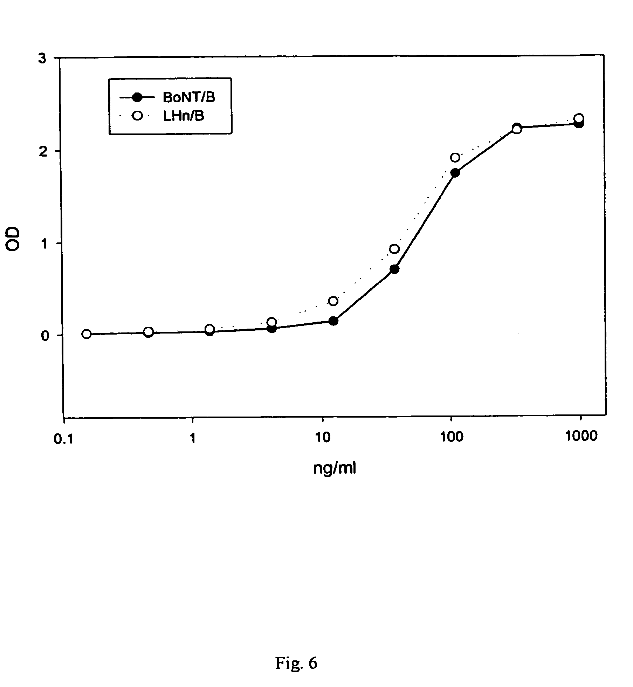

Referring to Fig. 6, dilutions of recLH_{N}/B (prepared as described in Example 4) and BoNT/B were compared in an *in vitro* peptide cleavage assay. Data indicate that the recombinant product has similar catalytic activity to that of the native neurotoxin, indicating that the recombinant product has folded correctly into an active conformation.

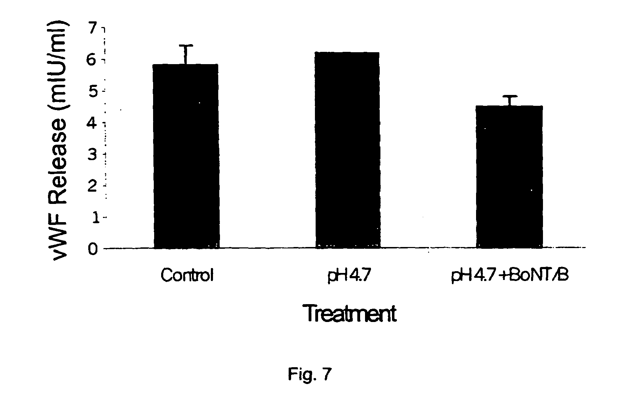

Referring to Fig. 7, cells were exposed to pH 4.7 media with or without 500nM BoNT/B (control cells received pH7.4 medium) for 2.5 hours then washed. 24 hours later release of vWF was stimulated using 1mM histamine and the presented results are the net stimulated release with basal subtracted. Results are presented in mIU of vWF/ml and are the mean ± SEM of three determinations apart from pH 4.7 alone which is two determinations. pH 4.7 + BoNT/B has reduced vWF release by 27.4% compared to pH 4.7 controls.

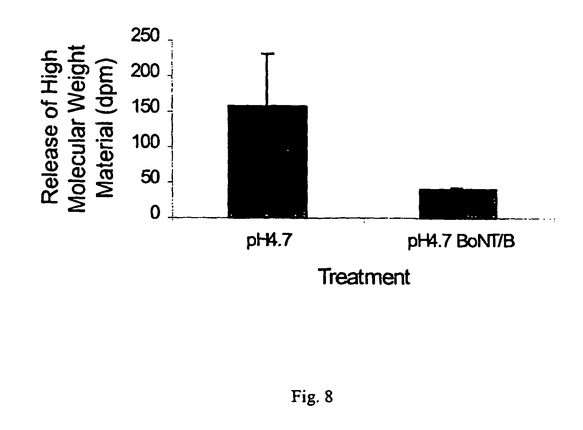

Referring to Fig. 8, high molecular weight mucin synthesising colon carcinoma LS180 cells were treated with pH 4.7 medium and pH 4.7 medium containing 500nM botulinum neurotoxin type B (BoNT/B) for four hours then labelled with [³H]-glucosamine for 18 hours. Release of high molecular weight material was stimulated with 10µM ionomycin and [³H]-glucosamine labelled material recovered by ultracentrifugation and centrifugal molecular weight sieving. Radiolabel of release of labelled high molecular weight material was determined by scintillation counting and net stimulated release calculated by subtracting non-stimulated basal values. Data are expressed as disintegrations per minute (dpm) ± SEM of three determinations. BoNT/B co-treatment clearly inhibits the release of high molecular weight material from these mucin synthesising cells and in this experiment a 74.5% reduction was seen.

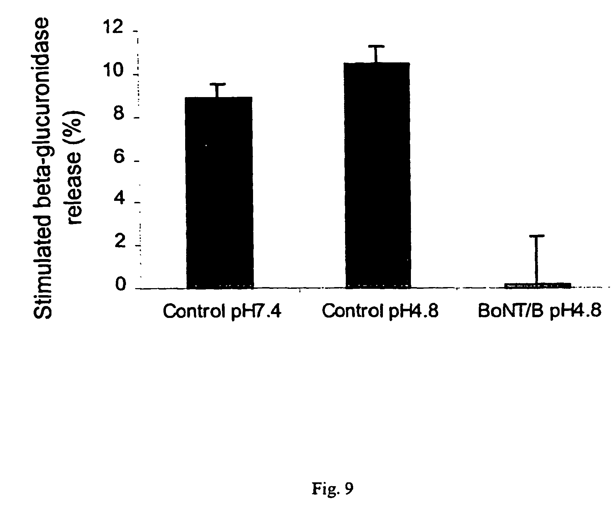

Referring to Fig. 9, cells were exposed to pH 4.8 media with or without 500nM BoNT/B (control cells received pH 7.4 medium) for 2.5 hours then washed and differentiated for 40 hours by the addition of 300µM dibutyryl cyclic AMP (dbcAMP). Cells were stimulated with fMet-Leu-Phe (1µM) + ATP (100µM) in the presence of cytochalasin B (5µM) for 10 minutes and released β-glucuronidase determined by colourimetric assay. Net stimulated release was calculated by subtraction of unstimulated basal release values from stimulated values and released activity is expressed as a percentage of the total activity present in the cells. Data are the mean ± SEM of three determinations. BoNT/B treatment in low pH medium significantly inhibited stimulated release of β-glucuronidase compared to cells treated with low pH alone (p=0.0315 when subjected to a 2 tailed Student T test with groups of unequal variance).

### Example 1

### Production of a conjugate of a lectin from Triticum vulgaris and LH_{N}/A

### Materials

Lectin from *Triticum vulgaris* (Wheat Germ Agglutinin - WGA) was obtained from Sigma Ltd.
SPDP was from Pierce Chemical Co.
PD-10 desalting columns were from Pharmacia.
Dimethylsulphoxide (DMSO) was kept anhydrous by storage over a molecular sieve.
Denaturing sodium dodecylsulphate polyacrylamide gel electrophoresis (SDS-PAGE) and non-denaturing polyacrylamide gel electrophoresis was performed using gels and reagents from Novex.
Additional reagents were obtained from Sigma Ltd.

LH_{N}/A was prepared according to a previous method (Shone, C. C. and Tranter, H. S. (1995) in "Clostridial Neurotoxins - The molecular pathogenesis of tetanus and botulism", (Montecucco, C., Ed.), pp. 152-160, Springer). FPLC^{®} chromatography media and columns were obtained from Amersham Pharmacia Biotech, UK. Affi-gel Hz™ matrix and materials were from BioRad, UK.

### Preparation of an anti-BoNT/A antibody-affinity column

An antibody-affinity column was prepared with specific monoclonal antibodies essentially as suggested by the manufacturers' protocol. Briefly, monoclonal antibodies 5BA2.3 & 5BA9.3 which have different epitope recognition in the H_{C} domain (Hallis, B., Fooks, S., Shone, C. and Hambleton, P. (1993) in "Botulinum and Tetanus Neurotoxins", (DasGupta, B. R., Ed.), pp. 433-436, Plenum Press, New York) were purified from mouse hybridoma tissue culture supernatant by Protein G (Amersham Pharmacia Biotech) chromatography. These antibodies represent a source of BoNT/A H_{C}-specific binding molecules and can be immobilised to a matrix or used free in solution to bind BoNT/A. In the presence of partially purified LH_{N}/A (which has no H_{C} domain) these antibodies will only bind to BoNT/A. The antibodies 5BA2.3 & 5BA9.3 were pooled in a 3:1 ratio and two mg of the pooled antibody was oxidised by the addition of sodium periodate (final concentration of 0.2%) prior coupling to 1 ml Affi-Gel Hz™ gel (16 hours at room temperature). Coupling efficiencies were routinely greater than 65%. The matrix was stored at 4 °C in the presence of 0.02% sodium azide.

### Purification strategy for the preparation of pure LH_{N}/A

BoNT/A was treated with 17µg trypsin per mg BoNT/A for a period of 72-120 hours. After this time no material of 150kDa was observed by SDS-PAGE and Coomassie blue staining. The trypsin digested sample was chromatographed (FPLC^{®}system, Amersham Pharmacia Biotech) on a Mono Q^{®} column (HR5/5) to remove trypsin and separate the majority of BoNT/A from LH_{N}/A. The crude sample was loaded onto the column at pH 7 in 20mM HEPES, 50mM NaCl and 2ml LH_{N}/A fractions eluted in a NaCl gradient from 50mM to 150mM. The slightly greater pl of BoNT/A (6.3) relative to LH_{N}/A (5.2) encouraged any BoNT/A remaining after trypsinisation to elute from the anion exchange column at a lower salt concentration than LH_{N}/A. LH_{N}/A containing fractions (as identified by SDS-PAGE) were pooled for application to the antibody column.

The semi-purified LH_{N}/A mixture was applied and reapplied at least 3 times to a 1-2ml immobilised monoclonal antibody matrix at 20 °C. After a total of 3 hours in contact with the immobilised antibodies, the LH_{N}/A-enriched supernatant was removed. Entrapment of the BoNT/A contaminant, rather than specifically binding the LH_{N}/A, enables the elution conditions to be maintained at the optimum for LH_{N} stability. The use of harsh elution conditions e.g. low pH, high salt, chaotropic ions, which may have detrimental effects on LH_{N} polypeptide folding and enzymatic activity, are therefore avoided. Treatment of the immobilised antibody column with 0.2M glycine/HCl pH2.5 resulted in regeneration of the column and elution of BoNT/A-reactive proteins of 150kDa.

The LH_{N}/A enriched sample was then applied 2 times to a 1 ml HiTrap^{®} Protein G column (Amersham Pharmacia Biotech) at 20 °C. Protein G was selected since it has a high affinity for mouse monoclonal antibodies. This step was included to remove BoNT/A-antibody complexes that may leach from the immunocolumn. Antibody species bind to the Protein G matrix allowing purified LH_{N}/A to elute, essentially by the method of Shone C.C., Hambleton, P., and Melling, J. 1987, Eur. J. Biochem. 167, 175-180, and as described in PCT/GB00/03519.

### Methods

The lyophilised lectin was rehydrated in phosphate buffered saline (PBS) to a final concentration of 10 mg/ml. Aliquots of this solution were stored at - 20 °C until use.

The WGA was reacted with an equal concentration of SPDP by the addition of a 10 mM stock solution of SPDP in DMSO with mixing. After one hour at room temperature the reaction was terminated by desalting into PBS over a PD-10 column.

The thiopyridone leaving group was removed from the product to release a free -SH group by reduction with dithiothreitol (DTT; 5 mM; 30 min). The thiopyridone and DTT were removed by once again desalting into PBS over a PD-10 column.

The LH_{N}/A was desalted into PBSE (PBS containing 1 mM EDTA). The resulting solution (0.5-1.0 mg/ml) was reacted with a four-fold molar excess of SPDP by addition of a 10 mM stock solution of SPDP in DMSO. After 3 h at room temperature the reaction was terminated by desalting over a PD-10 column into PBSE.

A portion of the derivatized LH_{N}/A was removed from the solution and reduced with DTT (5 mM, 30 min). This sample was analyzed spectrophotometrically at 280 nm and 343 nm to determine the degree of derivatisation. The degree of derivatisation achieved was 3.53 ± 0.59 mol/mol.

The bulk of the derivatized LH_{N}/A and the derivatized WGA were mixed in proportions such that the WGA was in greater than three-fold molar excess. The conjugation reaction was allowed to proceed for > 16 h at 4 °C.

The product mixture was centrifuged to clear any precipitate that had developed. The supernatant was concentrated by centrifugation through concentrators (with 10000 molecular weight exclusion limit) before application to a Superose 12 column on an FPLC chromatography system (Pharmacia). The column was eluted with PBS and the elution profile followed at 280 nm.

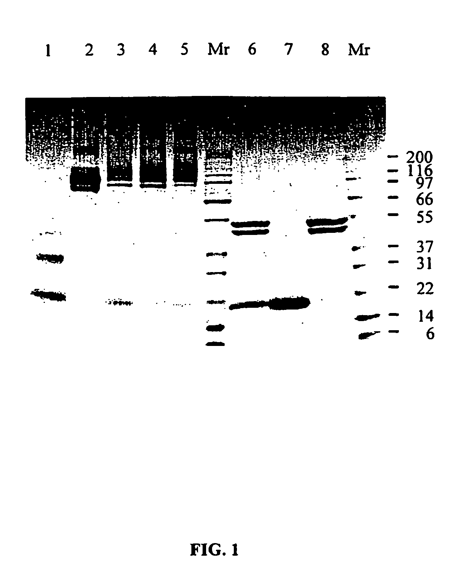

Fractions were analyzed by SDS-PAGE on 4-20% polyacrylamide gradient gels, followed by staining with Coomassie Blue. The major conjugate products have an apparent molecular mass of between 106-150 kDa, these are separated from the bulk of the remaining unconjugated LH_{N}/A and more completely from the unconjugated WGA. Fractions containing conjugate were pooled prior to addition to PBS-washed N-acetylglucosamine-agarose. Lectin-containing proteins (i.e. WGA-LH_{N}/A conjugate) remained bound to the agarose during washing with PBS to remove contaminants (predominantly unconjugated LH_{N}/A). WGA-LH_{N}/A conjugate was eluted from the column by the addition of 0.3M N-acetylglucosamine (in PBS) and the elution profile followed at 280 nm. See Fig 1 for SDS-PAGE profile of the whole purification scheme.

The fractions containing conjugate were pooled, dialysed against PBS, and stored at 4 °C until use.

### Example 2

### Activity of WGA-LH_{N}/A in cultured endocrine cells (HIT-T15)

The hamster pancreatic B cell line HIT-T15 is an example of a cell line of endocrine origin. It thus represents a model cell line for the investigation of inhibition of release effects of the agents. HIT-T15 cells possess surface moieties that allow for the binding, and internalisation, of WGA-LH_{N}/A.

In contrast, HIT-T15 cells lack suitable receptors for clostridial neurotoxins and are therefore not susceptible to botulinum neurotoxins (BoNTs).

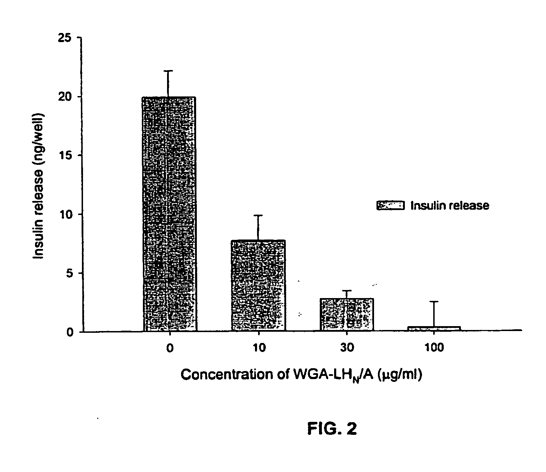

Fig. 2 illustrates the inhibition of release of insulin from HIT-T15 cells after prior incubation with WGA-LH_{N}/A. It is clear that dose-dependent inhibition is observed, indicating that WGA-LH_{N}/A can inhibit the release of insulin from an endocrine cell model.

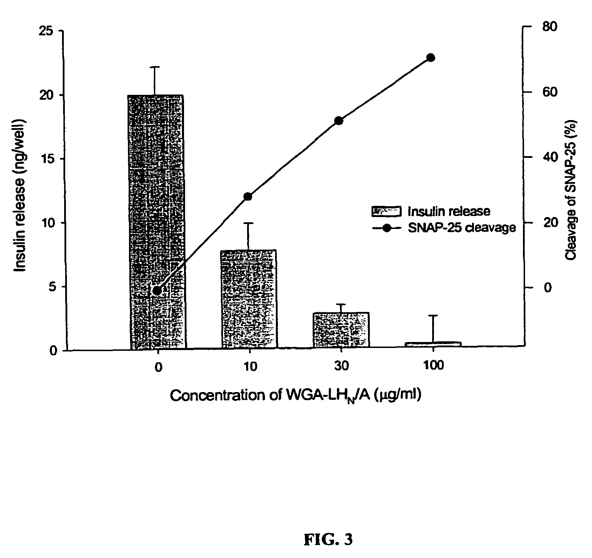

Inhibition of insulin release was demonstrated to correlate with cleavage of the SNARE protein, SNAP-25 (Fig. 3). Thus, inhibition of release of chemical messenger is due to a clostridial endopeptidase-mediated effects of SNARE-protein cleavage.

### Materials

### Insulin radioimmunoassay kits were obtained from Linco Research Inc., USA.

Western blotting reagents were obtained from Novex.

### Methods

HIT-T15 cells were seeded onto 12 well plates and cultured in RPMI-1640 medium containing 5% foetal bovine serum, 2mM L-glutamine for 5 days prior to use. WGA-LH_{N}/A was applied for 4 hours on ice, the cells were washed to remove unbound WGA-LH_{N}/A, and the release of insulin assayed 16 hours later. The release of insulin from HIT-T15 cells was assessed by radioimmunoassay exactly as indicated by the manufacturers' instructions.

Cells were lysed in 2M acetic acid / 0.1% TEA. Lysates were dried then resuspended in 0.1 M Hepes, pH 7.0. To extract the membrane proteins Triton-X-114 (10%, v/v) was added and incubated at 4°C for 60 min. The insoluble material was removed by centrifugation and the supernatants were warmed to 37°C for 30 min. The resulting two phases were separated by centrifugation and the upper phase discarded. The proteins in the lower phase were precipitated with chloroform/methanol for analysis by Western blotting.

The samples were separated by SDS-PAGE and transferred to nitrocellulose. Proteolysis of SNAP-25, a crucial component of the neurosecretory process and the substrate for the zinc-dependent endopeptidase activity of BoNT/A, was then detected by probing with an antibody (SMI-81) that recognises both the intact and cleaved forms of SNAP-25.

### Example 3

### Activity of WGA-LH_{N}/A in cultured neuroendocrine cells (PC12)

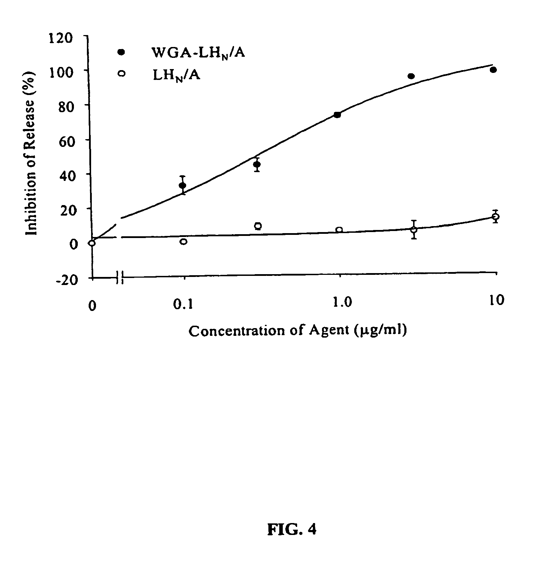

The rat pheochromocytoma PC12 cell line is an example of a cell line of neuroendocrine origin. In its undifferentiated form it has properties associated with the adrenal chromaffin cell [Greene and Tischler, in "Advances in Cellular Neurobiology" (Federoff and Hertz, eds), Vol. 3, p373-414. Academic Press, New York, 1982]. It thus represents a model cell line for the investigation of inhibition of release effects of the agents. PC12 cells possess surface moieties that allow for the binding, and internalisation, of WGA-LH_{N}/A. Figure 4 illustrates the inhibition of release of noradrenaline from PC12 cells after prior incubation with WGA-LH_{N}/A. It is clear that dose-dependent inhibition is observed, indicating that WGA-LH_{N}/A can inhibit the release of hormone from a neuroendocrine cell model. Comparison of the inhibition effects observed with conjugate and the untargeted LH_{N}/A demonstrate the requirement for a targeting moiety (TM) for efficient inhibition of transmitter release.

### Methods

PC12 cells were cultured on 24 well plates in RPMI-1640 medium containing 10% horse serum, 5% foetal bovine serum, 1% L-glutamine. Cells were treated with a range of concentrations of WGA-LH_{N}/A for three days. Secretion of noradrenaline was measured by labelling cells with [^{3\}H]-noradrenaline (2µCi/ml, 0.5ml/well) for 60 min. Cells were washed every 15 min for 1 hour then basal release determined by incubation with a balanced salt solution containing 5mM KCI for 5 min. Secretion was stimulated by elevating the concentration of extracellular potassium (100mM KCl) for 5 min. Radioactivity in basal and stimulated superfusates was determined by scintillation counting. Secretion was expressed as a percentage of the total uptake and stimulated secretion was calculated by subtracting basal. Inhibition of secretion was dose-dependent with an observed IC₅₀ of 0.63 ± 0.15µg/ml (n = 3). Inhibition was significantly more potent when compared to untargeted endopeptidase (LH_{N}/A in Fig. 4). Thus WGA-LH_{N}/A inhibits release of neurotransmitter from a model neuroendocrine cell type.

### Example 4

### Expression and purification of catalytically active recombinant LH_{N}/B

The coding region for LH_{N}/B was inserted in-frame to the 3' of the gene encoding maltose binding protein (MBP) in the expression vector pMAL (New England Biolabs). In this construct, the expressed MBP and LH_{N}/B polypeptides are separated by a Factor Xa cleavage site.

Expression of the MBP-LH_{N}/B in *E. coli* TG1 was induced by addition of IPTG to the growing culture at an approximate OD600nm of 0.8. Expression was maintained for a further 3 hours in the presence of inducing agent prior to harvest by centrifugation. The recovered cell paste was stored at -20°C until required.

The cell paste was resuspended in resuspension buffer (50mM Hepes pH7.5 + 150mM NaCl⁺ a variety of protease inhibitors) at 6ml buffer per gram paste. To this suspension was added lysozyme to a final concentration of 1mg/ml. After 10 min at 0°C, the suspension was sonicated for 6x30 seconds at 24µ at 0°C. The broken cell paste was then centrifuged to remove cell debris and the supernatant recovered for chromatography.

In some situations, the cell paste was disrupted by using proprietary disruption agents such as BugBuster™ (Novagen) as per the manufacturers protocol. These agents were satisfactory for disruption of the cells to provide supernatant material for affinity chromatography.

The supernatant was applied to an immobilised amylose matrix at 0.4ml/min to facilitate binding of the fusion protein. After binding, the column was washed extensively with resuspension buffer to remove contaminating proteins. Bound proteins were eluted by the addition of elution buffer (resuspension buffer + 10mM maltose) and fractions collected. Eluted fractions containing protein were pooled for treatment with Factor Xa.

On some occasions a further purification step was incorporated into the scheme, prior to the addition of Factor Xa. In these instances, the eluted fractions were made to 5mM DTT and applied to a Pharmacia Mono-Q HR5/5 column (equilibrated in resuspension buffer) as part of an FPLC system. Proteins were bound to the column at 150mM NaCl, before increased to 500mM NaCl over a gradient. Fractions were collected and analysed for the presence of MBP-LH_{N}/B by Western blotting (probe antibody = guinea pig anti-BoNT/B or commercially obtained anti-MBP).

Cleavage of the fusion protein by Factor Xa was as described in the protocol supplied by the manufacturer (New England Biolabs). Cleavage of the fusion protein resulted in removal of the MBP fusion tag and separation of the LC and H_{N} domains of LH_{N}/B. Passage of the cleaved mixture through a second immobilised maltose column removed free MBP from the mixture to leave purified disulphide-linked LH_{N}/B. This material was used for conjugation.

See Figure 5 for an illustration of the purification of LH_{N}/B.

See Figure 6 for an illustration of the *in vitro* catalytic activity of LH_{N}/B.

### Example 5

### Production of a conjugate of a lectin from Triticum vulgaris and LH_{N}/B

### Materials

Lectin from *Triticum vulgaris* (WGA) was obtained from Sigma Ltd. LH_{N}/B was prepared as described in Example 4.
SPDP was from Pierce Chemical Co.
PD-10 desalting columns were from Pharmacia.
Dimethylsulphoxide (DMSO) was kept anhydrous by storage over a molecular sieve.
Polyacrylamide gel electrophoresis was performed using gels and reagents from Novex.
Additional reagents were obtained from Sigma Ltd.

### Methods

The lyophilised lectin was rehydrated in phosphate buffered saline (PBS) to a final concentration of 10 mg/ml. Aliquots of this solution were stored at - 20 ° C until use.

The WGA was reacted with an equal concentration of SPDP by the addition of a 10 mM stock solution of SPDP in DMSO with mixing. After one hour at room temperature the reaction was terminated by desalting into PBS over a PD-10 column.

The thiopyridone leaving group was removed from the product to release a free -SH group by reduction with dithiothreitol (DTT; 5 mM; 30 min). The thiopyridone and DTT were removed by once again desalting into PBS over a PD-10 column.

The *rec*LH_{N}/B was desalted into PBS. The resulting solution (0.5-1.0 mg/ml) was reacted with a four-fold molar excess of SPDP by addition of a 10 mM stock solution of SPDP in DMSO. After 3 h at room temperature the reaction was terminated by desalting over a PD-10 column into PBS.

A portion of the derivatized *rec*LH_{N}/B was removed from the solution and reduced with DTT (5 mM, 30 min). This sample was analysed spectrophotometrically at 280 nm and 343 nm to determine the degree of derivatisation.

The bulk of the derivatized *rec*LH_{N}/B and the derivatized WGA were mixed in proportions such that the WGA was in greater than three-fold molar excess. The conjugation reaction was allowed to proceed for > 16 h at 4 °C.

The product mixture was centrifuged to clear any precipitate that had developed. The supernatant was concentrated by centrifugation through concentrators (with 10000 molecular weight exclusion limit) before application to a Superdex G-200 column on an FPLC chromatography system (Pharmacia). The column was eluted with PBS and the elution profile followed at 280 nm.

Fractions were analysed by SDS-PAGE on 4-20% polyacrylamide gradient gels, followed by staining with Coomassie Blue. The major conjugate products have an apparent molecular mass of between 106-150 kDa, these are separated from the bulk of the remaining unconjugated *rec*LH_{N}/B and more completely from the unconjugated WGA. Fractions containing conjugate were pooled prior to addition to PBS-washed N-acetylglucosamine-agarose. Lectin-containing proteins (i.e. WGA-*rec*LH_{N}/B conjugate) remained bound to the agarose during washing with PBS to remove contaminants (predominantly unconjugated *rec*LH_{N}/B). WGA-*rec*LH_{N}/B conjugate was eluted from the column by the addition of 0.3M N-acetylglucosamine (in PBS) and the elution profile followed at 280 nm.

The fractions containing conjugate were pooled, dialysed against PBS, and stored at 4 °C until use.

### Example 6

### Activity of BoNT/B in vascular endothelial cells

Human umbilical vein endothelial cells (HUVEC) secrete von Willebrands Factor (vWF) when stimulated with a variety of cell surface receptor agonists including histamine. These cells maintain this property when prepared from full term umbilical cords and grown in culture (Loesberg et al 1983, Biochim. Biophys. Acta. 763, 160-168). The release of vWF by HUVEC thus represents a secretory activity of a non-neuronal cell type derived from the cardiovascular system. Fig. 7 illustrates the inhibition of the histamine stimulated release of vWF by HUVEC when previously treated with BoNT/B in low pH medium. Treatment of cells with toxins in low pH can be used as a technique for facilitating toxin penetration of the plasmalemma of cells refractory to exogenously applied clostridial neurotoxins.

This result clearly shows the ability of botulinum neurotoxins to inhibit secretory activity of non-neuronal cells in the cardiovascular system (see Fig. 7).

### Methods

HUVEC were prepared by the method of Jaffe et al 1973, J. Clin. Invest. 52, 2745-2756. Cells were passaged once onto 24 well plates in medium 199 supplemented with 10% foetal calf serum, 10% newborn calf serum, 5mM L-glutamine, 100 units/ml penicillin, 100 units/ml streptomycin, 20µg/ml endothelial cell growth factor (Sigma). Cells were treated with DMEM pH 7.4, DMEM pH 4.7 (pH lowered with HCl) or DMEM, pH 4.7 with 500nM BoNT/B for 2.5 hours then washed three times with HUVEC medium. 24 hours later cells were washed with a balanced salt solution, pH 7.4 and exposed to this solution for 30 minutes for the establishment of basal release. This was removed and BSS containing 1 mM histamine applied for a further 30 minutes. Superfusates were centrifuged to remove any detached cells and the quantity of vWF determined using an ELISA assay as described by Paleolog et al 1990, Blood. 75, 688-695. Stimulated secretion was then calculated by subtracting basal from the histamine stimulated release. Inhibition by BoNT/B treatment at pH 4.7 was calculated at 27.4% when compared to pH 4.7 treatment alone.

### Example 7

### Activity of BoNT/B in mucus secreting cells

The LS180 colon carcinoma cell line is recognised as a model of mucin secreting cells (McCool, D. J., Forstner, J. F. and Forstner, G. G. 1994 Biochem. J. 302, 111-118). These cells have been shown to adopt goblet cell morphology and release high molecular weight mucin when stimulated with muscarinic agonists (eg carbachol), phorbol esters (PMA) and Ca²⁺ ionophores (eg A23187) (McCool, D. J., Forstner, J. F. and Forstner, G. G. 1995 Biochem. J. 312, 125-133). These cells thus represent a non-neuronal cell type derived from the colon which can undergo regulated mucin secretion. Fig. 8 illustrates the inhibition of the ionomycin stimulated release of high molecular weight, [³H]-glucosamine labelled material from LS180 cells by pretreatment with BoNT/B in low pH medium. Ionomycin is a Ca²⁺ ionophore and treatment of cells with low pH medium has been previously shown to facilitate toxin entry into cells.

This result clearly shows the ability of botulinum neurotoxins to inhibit secretory activity of non-neuronal cells able to release mucin when stimulated with a secretagogue (see Fig. 8).

### Methods

Mucin synthesising colon carcinoma LS180 cells were grown on Matrigel coated 24 well plates in minimum essential medium supplemented with 10% foetal calf serum, 2mM L-glutamine and 1 % non-essential amino acids (Sigma) Cells were treated with pH 7.4 medium, pH 4.7 medium and pH 4.7 medium containing 500nM botulinum neurotoxin type B (BoNT/B) for four hours then labelled with [³H]-glucosamine (1µCi/ml, 0.5ml/well) for 18 hours in L15 glucose free medium. Cells were then washed twice with a balanced salt solution (BSS) pH 7.4 and then 0.5ml of BSS was applied for 30 minutes. This material was removed and 0.5ml of BSS containing 10µM ionomycin applied to stimulate mucin release. The stimulating solution was removed and all superfusates centrifuged to remove any detached cells. Supernatants were then centrifuged at 100,000 x g for 1 hour. Supernatants were applied to Centricon centrifugal concentrators with a molecular weight cut-off of 100kDa and centrifuged (2,500 x g) until all liquid had passed through the membrane. Membranes were washed with BSS by centrifugation three times and then the membrane scintillation counted for retained, [³H]-glucosamine labelled high molecular weight material.

### Example 8

### Activity of BoNT/B in inflammatory cells

The promyelocytic cell line HL60 can be differentiated into neutrophil like cells by the addition of dibutyryl cyclic AMP to the culture medium. Upon differentiation these cells increase their expression of characteristic enzymes such as β-glucuronidase. In this condition these cells therefore represent a model of a phagocytic cell type which contributes to the inflammatory response of certain disease states (eg rheumatoid arthritis). Figure 9 illustrates the significant (p > 0.05) inhibition of stimulated release of β-glucuronidase from dbcAMP differentiated HL60 cells by pre-treatment with BoNT/B in low pH medium.

This result clearly shows the ability of botulinum neurotoxins to inhibit the secretory activity of a non-neuronal cell type which is a model of the neutrophil a cell which participates in inflammation.

### Methods

HL60 cells were cultured in RPMI 1640 medium containing 10% foetal calf serum and 2mM glutamine. Cells were exposed to low pH and toxin for 2.5 hours then washed 3 times and differentiated by the addition of dibutyryl cyclic AMP (dbcAMP) to a final concentration of 300µM. Cells were differentiated for 40 hours and then stimulated release of β-glucuronidase activity was determined. Cells were treated with cytochalasin B (5µM) 5 minutes before stimulation. Cells were stimulated with 1µM N-formyl-Met-Leu-Phe with 100µM ATP for 10 minutes then centrifuged and the supernatant taken for assay of β-glucuronidase activity. Activity was measured in cell lysates and the amount released expressed as a percentage of the total cellular content of enzyme.

β-glucuronidase activity was determined according to the method of Absolom D.R. 1986, (Methods in Enzymology, 132, 160) using *p-*Nitrophenyl-β-D-glucuronide as the substrate.

## Claims

1. Use of an agent for the manufacture of a medicament for inhibiting secretion from a non-neuronal inflammatory cell, said agent comprising first, second and third domains
wherein the first domain comprises a clostridial neurotoxin light chain or a fragment thereof that cleaves one or more proteins essential to exocytosis,
the second domain comprises a translocation domain selected from the group consisting of a clostridial toxin heavy chain H_{N} portion, a diphtheria toxin translocation domain, a pseudomonas exotoxin A translocation domain, an influenza virus haemagglutinin fusogenic translocation domain, an amphiphilic peptide translocation domain, and fragments thereof that translocate the first domain into the inflammatory cell,
and the third domain comprises a Targeting Moiety that binds with high specificity and/ or affinity to a Binding Site on the surface of said non-neuronal inflammatory cell, wherein the Targeting Moiety is selected from the group consisting of antibodies, monoclonal antibodies, antibody fragments, lectins, hormones, cytokines, growth factors, peptides, carbohydrates, lipide, glycons, and complement components,

2. Use according to Claim 1, for treatment of disease caused, exacerbated or maintained by secretion from said non-neuronal inflammatory cell.

3. Use according to Claim 1 wherein the third domain comprises or consists of a ligand selected from (i) for mast cells, complement receptors in general, including C4 domain of the Fc IgE, and the antibodies/ligands to the C3a/C4a-R complement receptor; (ii) for eosinophils, antibodies/ligands to the C3a/C4a-R complement receptor, anti VLA-4 monoclonal antibody, anti-IL5 receptor, antigens or antibodies reactive toward CR4 complement receptor (iii) for macrophages and monocytes, macrophage stimulating factor, (iv) for macrophages, monocytes and neutrophils, bacterial LPS and yeast B-glucans which bind to CR3, (v) for neutrophils, antibody to OX42, an antigen associated with the iC3b complement receptor, or IL8; (vi) for fibroblasts, mannose 6-phosphate/insulin-like growth factor-beta (M6P/IGP-II) receptor and PA2.26, antibody to a cell-surface receptor for active fibroblasts in mice; and (vii) growth factors.

4. Use according to any previous Claim for the treatment of a disease selected from the group consisting of allergies (seasonal allergic rhinitis (hay fever), allergic conjunctivitis, vasomotor rhinitis and food allergy), eosinophilia, asthma, rheumatoid arthritis, systemic lupus erythematosus, discoid lupus erythematosus, ulcerative colitis, Crohn's disease, haemorrhoids, pruritus, glomerulonephritis, hepatitis, pacreatitis, gastritis, vasculitis, myocarditis, psoriasis, eczema, chronic radiation-induced fibrosis, lung scarring and other fibrotic disorders.

5. Use according to any previous Claim, wherein the agent comprises a first domain that cleaves a protein selected from SNAP-25, synaptobrevin and syntaxin.

6. Use according to any previous Claim, wherein the second domain comprises a H_{N} region of a clostridial polypeptide or a fragment thereof that translocates the exocytosis inhibiting activity of the first domain into the cell.

7. Use according to any previous Claim for inhibition of constitutive and regulated release from non-neuronal inflammatory cells.

8. An agent for inhibiting secretion from a non-neuronal inflammatory cell, comprising at least first, second and third domains
wherein the first domain comprises a clostridial neurotoxin light chain or a fragment thereof that cleaves one or more proteins essential to exocytosis,
the second domain comprises a translocation domain selected from the group consisting of a clostridial toxin heavy chain H_{N} portion, a diphtheria toxin translocation domain, a pseudomonas exotoxin A translocation domain, an influenza virus haemagglutinin fusogenic translocation domain, an amphiphilic peptide translocation domain, and fragments thereof that translocate the first domain into the cell
and the third domain comprises a Targeting Moiety that binds with high specificity and/ or affinity to a Binding Site on the surface of a non-neuronal inflammatory cell, wherein the Targeting Moiety is selected from the group consisting of antibodies, monoclonal antibodies, antibody fragments, lectins, hormones, cytokines, growth factors, peptides, carbohydrates, lipids, glycons, and complement components.

9. An agent according to Claim 8, wherein the third domain is as defined in Claim 3.

10. A pharmaceutical composition comprising an agent according to Claim 8 or 9 in combination with a pharmaceutically acceptable carrier.

11. Use of an agent according to Claim 8 or 9 or a pharmaceutical composition according to Claim 10, for the manufacture of a medicament for treatment of a disease caused, exacerbated or maintained by secretion from a non-neuronal inflammatory cell.

12. A nucleic acid construct encoding an agent according to Claim 8 or 9, said construct comprising nucleic acid sequences encoding the first, second and third domains.

13. A nucleic acid construct according to Claim 12, operably linked to promoter and terminator sequences, and optionally regulatory sequences, said promoter, terminator and regulatory sequences being functional in a non-neuronal inflammatory target cell to effect expression of said agent in said target cell.

14. An agent for use in gene therapy, comprising:
(i) a nucleic acid sequence encoding a first domain comprises a clostridial neurotoxin light chain or a fragment thereof that cleaves one or more proteins essential to exocytosis;
(ii) a second domain associated with the nucleic acid sequence, wherein said second domain comprises a translocation domain selected from the group consisting of a clostridial toxin heavy chain H_{N} portion, a diphtheria toxin translocation domain, a pseudomonas exotoxin A translocation domain, an influenza virus haemagglutinin fusogenic translocation domain, an amphiphilic peptide translocation domain, and fragments thereof, which, following administration of the agent to a patient, translocate the nucleic acid sequence into a non-neuronal inflammatory target cell, wherein when in said non-neuronal inflammatory target cell, expression of the nucleic acid sequence is effected therein; and
(iii) a third domain comprises a Targeting Moiety that binds with high specificity and/ or affinity to a Binding Site on the surface for targeting the agent to said non-neuronal inflammatory cell, wherein the Targeting Moiety is selected from the group consisting of antibodies, monoclonal antibodies, antibody fragments, lectins, hormones, cytokines, growth factors, peptides, carbohydrates, lipids, glycons, and complement components.

15. An agent according to Claim 14, wherein the nucleic acid sequence is operably linked to promoter and terminator sequences, and optionally regulatory sequences, said promoter, terminator and regulatory sequences being functional in the non-neuronal inflammatory target cell to effect expression of said agent in said non-neuronal inflammatory target cell.

16. An agent according to Claim 14 or 15, wherein the third domain is as defined in Claim 3.

17. Use of an agent according to any of Claim 14-16 for the manufacture of a medicament for treating a disease caused, exacerbated or maintained by secretion from a non-neuronal inflammatory cell.

18. Use of a nucleic acid construct according to Claim 12 or 13 for the manufacture of a medicament for treating a disease caused by, exacerbated, or maintained by secretion from a non-neuronal inflammatory cell.

## Patentansprüche

1. Verwendung eines Wirkstoffs für die Herstellung von einem Medikament für die Inhibierung von Sekretion aus einer nicht-neuronalen Entzündungszelle, wobei der Wirkstoff eine erste, zweite und dritte Domäne umfasst,
wobei die erste Domäne eine leichte Kette von Clostridien-Neurotoxin oder ein Fragment davon, das ein oder mehrere für die Exocytose wesentliche Proteine spaltet, umfasst,
die zweite Domäne eine Translokationsdomäne, ausgewählt aus der Gruppe, bestehend aus einem H_{N}-Teil von einer schweren Kette von Clostridien-Toxin, einer Translokationsdomäne von Diphtherie-Toxin, einer Translokationsdomäne von Pseudomonas-Exotoxin A, einer fusogenen Translokationsdomäne von Hämagglutinin des Influenzavirus, einer amphiphilen Peptid-Translokationsdomäne sowie Fragmenten davon, welche die erste Domäne in die Entzündungszelle translozieren, umfasst,
und die dritte Domäne einen Targeting-Rest, der mit hoher Spezifität und/oder Affinität an eine Bindungsstelle auf der Oberfläche der nicht-neuronalen Entzündungszelle bindet, umfasst, wobei der Targeting-Rest aus der Gruppe, bestehend aus Antikörpern, monoklonalen Antikörpern, Antikörperfragmenten, Lektinen, Hormonen, Cytokinen, Wachstumsfaktoren, Peptiden, Kohlenhydraten, Lipiden, glycons und Bestandteilen des Komplementsystems, ausgewählt ist.

2. Verwendung nach Anspruch 1 für die Behandlung einer Krankheit, verursacht, verschlimmert oder aufrechterhalten durch Sekretion aus den nicht-neuronalen Entzündungszellen.

3. Verwendung nach Anspruch 1, wobei die dritte Domäne einen Liganden, ausgewählt aus (i) für Mastzellen, Komplementrezeptoren allgemein, einschließlich der C4-Domäne von dem Fc IgE, und den Antikörper/Liganden zu dem C3a/C4a-R-Komplementrezeptor; (ii) für Eosinophile, Antikörpern/Liganden zu dem C3a/C4a-R-Komplementrezeptor, anti-VLA-4 monoklonalem Antikörper, anti-IL5-Rezeptor, gegenüber CR4-Komplementrezeptor reaktiven Antigenen oder Antikörpern; (iii) für Makrophagen, Monozyten und Makropagen-Anregungsfaktor, (iv) für Makrophagen, Monozyten und Neutrophilen, bakteriellen LPS und B-Glucanen von Hefe, die an CR3 binden, (v) für Neutrophile, Antikörpern zu OX42, einem mit dem iC3b-Komplementrezeptor assoziierten Antigen oder IL8; (vi) für Fibroblasten, Mannose-6-phosphat/Insulin-artigem-Wachstumsfaktor-beta(M6P/IGF-II)-Rezeptor und PA2.26, Antikörper zu einem Zelloberfläche-Rezeptor für aktive Fibroblasten in Mäusen; und (vii) Wachstumsfaktoren, umfasst oder daraus besteht.

4. Verwendung nach einem vorhergehenden Anspruch zur Behandlung einer Krankheit, ausgewählt aus der Gruppe, bestehend aus Allergien (saisonale allergische Rhinitis (Heuschnupfen), allergische Bindehautentzündung, vasomotorische Rhinitis und Lebensmittelallergie), Eosinophilie, Asthma, rheumatoide Arthritis, systemischer Lupus erythematosus, discoider Lupus erythematosus, Colitis ulcerosa, Morbus Crohn, Hämorrhoiden, Pruritus, Glomerulonephritis, Hepatitis, Pancreatitis, Gastritits, Vasculitis, Myocarditis, Psoriasis, Ekzem, chronische strahlungsinduzierte Fibrose, Lungenvernarbung und andere fibrotische Krankheiten.

5. Verwendung nach einem vorhergehenden Anspruch, wobei der Wirkstoff eine erste Domäne, die ein aus SNAP-25, Synaptobrevin und Syntaxin ausgewähltes Protein spaltet, umfasst.

6. Verwendung nach einem vorhergehenden Anspruch, wobei die zweite Domäne einen H_{N}-Teil von einem Clostridien-Polypeptid oder ein Fragment davon, der die Exocytose-inhibierende Aktivität von der ersten Domäne in die Zelle transloziert, umfasst.

7. Verwendung nach einem vorhergehenden Anspruch zur Inhibierung von konstitutiver und regulierter Ausschüttung aus nicht-neuronalen Entzündungszellen.

8. Wirkstoff zur Inhibierung von Sekretion aus einer nicht-neuronalen Entzündungszelle, umfassend mindestens eine erste, zweite und dritte Domäne,
wobei die erste Domäne eine leichte Kette von Clostridien-Neurotoxin oder ein Fragment davon, das ein oder mehrere für die Exocytose wesentliche Proteine spaltet, umfasst,
die zweite Domäne eine Translokationsdomäne, ausgewählt aus der Gruppe, bestehend aus einem H_{N}-Teil von einer schweren Kette von Clostridien-Toxin, einer Translokationsdomäne von Diphtherie-Toxin, einer Translokationsdomäne von Pseudomonas-Exotoxin A, einer fusogenen Translokationsdomäne von Hämagglutinin des Influenzavirus, einer amphiphilen Peptid-Translokationsdomäne sowie Fragmenten davon, welche die erste Domäne in die Zelle translozieren, umfasst,
und die dritte Domäne einen Targeting-Rest, der mit hoher Spezifität und/oder Affinität an eine Bindungsstelle auf der Oberfläche von einer nicht-neuronalen Entzündungszelle bindet, umfasst, wobei der Targeting-Rest aus der Gruppe, bestehend aus Antikörpern, monoklonalen Antikörpern, Antikörperfragmenten, Lektinen, Hormonen, Cytokinen, Wachstumsfaktoren, Peptiden, Kohlenhydraten, Lipiden, glycons und Bestandteilen des Komplementsystems, ausgewählt ist.

9. Wirkstoff nach Anspruch 8, wobei die dritte Domäne so ist, wie in Anspruch 3 definiert.

10. Pharmazeutische Zusammensetzung, umfassend einen Wirkstoff nach Anspruch 8 oder 9 in Kombination mit einem pharmazeutisch verträglichen Träger.

11. Verwendung eines Wirkstoffs nach Anspruch 8 oder 9 oder einer pharmazeutischen Zusammensetzung nach Anspruch 10 für die Herstellung von einem Medikament für die Behandlung von einer Krankheit, verursacht, verschlimmert oder aufrechterhalten durch Sekretion aus einer nicht-neuronalen Entzündungszelle.

12. Ein Nukleinsäurekonstrukt, kodierend einen Wirkstoff nach Anspruch 8 oder 9, wobei das Konstrukt Nukleinsäuresequenzen umfasst, welche die erste, zweite und dritte Domäne kodieren.

13. Nukleinsäurekonstrukt nach Anspruch 12, funktionsbereit verknüpft mit Promotor- und Terminatorsequenzen und wahlweise regulatorischen Sequenzen, wobei die Promotor-, Terminator- und regulatorischen Sequenzen in einer nicht-neuronalen Target-Entzündungszelle dahingehend funktionsfähig sind, die Exprimierung von dem Wirkstoff in der Target-Zelle zu bewirken.

14. Wirkstoff zur Verwendung in der Gentherapie, umfassend:
(i) eine Nukleinsäuresequenz, kodierend eine erste Domäne, die eine leichte Kette von Clostridien-Neurotoxin oder ein Fragment davon, das ein oder mehrere für die Exocytose wesentliche Proteine spaltet, umfasst;
(ii) eine zweite Domäne, die mit der Nukleinsäuresequenz assoziiert ist, wobei die zweite Domäne eine Translokationsdomäne, ausgewählt aus der Gruppe, bestehend aus einem H_{N}-Teil von einer schweren Kette von Clostridien-Toxin, einer Translokationsdomäne von Diphtherie-Toxin, einer Translokationsdomäne von Pseudomonas-Exotoxin A, einer fusogenen Translokationsdomäne von Hämagglutinin des Influenzavirus, einer amphiphilen Peptid-Translokationsdomäne sowie Fragmenten davon umfasst, welche, nachdem der Wirkstoff einem Patienten verabreicht wurde, die Nukleinsäuresequenz in eine nicht-neuronale Entzündungszelle translozieren, wobei, wenn innerhalb der nicht-neuronalen Target-Entzündungszelle, darin die Exprimierung von der Nukleinsäuresequenz bewirkt wird; und
(iii) eine dritte Domäne, umfassend einen Targeting-Rest, der mit hoher Spezifität und/oder Affinität an eine Bindungsstelle auf der Oberfläche, für das Targeting des Wirkstoffs zu der nicht-neuronalen Entzündungszelle, bindet, wobei der Targeting-Rest aus der Gruppe, bestehend aus Antikörpern, monoklonalen Antikörpern, Antikörperfragmenten, Lektinen, Hormonen, Cytokinen, Wachstumsfaktoren, Peptiden, Kohlenhydraten, Lipiden, glycons und Bestandteilen des Komplementsystems, ausgewählt ist.

15. Wirkstoff nach Anspruch 14, wobei die Nukleinsäuresequenz funktionsbereit mit Promotor- und Terminatorsequenzen und wahlweise regulatorischen Sequenzen verknüpft ist, wobei die Promotor-, Terminator- und regulatorischen Sequenzen in einer nicht-neuronalen Entzündungszelle dahingehend funktionsfähig sind, die Exprimierung von dem Wirkstoff in der nicht-neuronalen Target-Entzündungszelle zu bewirken.

16. Wirkstoff nach Anspruch 14 oder 15, wobei die dritte Domäne so wie in Anspruch 3 definiert ist.

17. Verwendung eines Wirkstoffs nach einem der Ansprüche 14 bis 16 für die Herstellung von einem Medikament für die Behandlung von einer Krankheit, verursacht, verschlimmert oder aufrechterhalten durch Sekretion aus einer nicht-neuronalen Entzündungszelle.

18. Verwendung von einem Nukleinsäurekonstrukt nach Anspruch 12 oder 13 für die Herstellung von einem Medikament für die Behandlung von einer Krankheit, verursacht, verschlimmert oder aufrechterhalten durch Sekretion aus einer nicht-neuronalen Entzündungszelle.

## Revendications

1. Utilisation d'un agent pour la fabrication d'un médicament pour inhiber une sécrétion d'une cellule inflammatoire non neuronale, ledit agent comprenant un premier, un deuxième et un troisième domaine
où le premier domaine comprend une chaîne légère de neurotoxine clostridiale ou un fragment de celle-ci qui clive une ou plusieurs protéines essentielles à l'exocytose,
le deuxième domaine comprend un domaine de translocation sélectionné dans le groupe consistant en une partie H_{N} de chaîne lourde de toxine clostridiale, un domaine de translocation de toxine diphtérique, un domaine de translocation de l'exotoxine A de Pseudomonas, un domaine de translocation fusogénique de l'hémagglutinine du virus de la grippe, un domaine de translocation de peptide amphiphile, et des fragments de ceux-ci qui entraînent la translocation du premier domaine dans la cellule inflammatoire,
et le troisième domaine comprend un fragment de ciblage qui se lie avec une haute spécificité et/ou affinité à un site de liaison sur la surface de ladite cellule inflammatoire non neuronale, où le fragment de ciblage est sélectionné dans le groupe consistant en des anticorps, des anticorps monoclonaux, des fragments d'anticorps, des lectines, des hormones, des cytokines, des facteurs de croissance, des peptides, des glucides, des lipides, des glycones, et des composants du complément.

2. Utilisation selon la revendication 1, pour le traitement d'une maladie provoquée, exacerbée ou maintenue par une sécrétion à partir de ladite cellule inflammatoire non neuronale.

3. Utilisation selon la revendication 1, dans laquelle le troisième domaine comprend ou consiste en un ligand sélectionné parmi (i) pour des mastocytes, des récepteurs du complément en général, comprenant le domaine C4 du Fc d'une IgE, et les anticorps/ligands dirigés contre le récepteur du complément C3a/C4a-R ; (ii) pour des éosinophiles, des anticorps/ligands dirigés contre le récepteur du complément C3a/C4a-R, un anticorps monoclonal anti-VLA-4, un anti-récepteur de l'IL5, des antigènes ou des anticorps réagissant avec le récepteur du complément CR4 ; (iii) pour des macrophages et des monocytes, le facteur stimulant les macrophages, (iv) pour des macrophages, monocytes et neutrophiles, un LPS bactérien et des B-glucanes de levure qui se lient à CR3, (v) pour des neutrophiles, un anticorps dirigé contre OX42, un antigène associé au récepteur du complément iC3b, ou l'IL-8 ; (vi) pour des fibroblastes, le récepteur du mannose 6-phosphate/facteur de croissance bêta analogue à l'insuline (M6P/IGF-II) et PA2.26, un anticorps dirigé contre un récepteur de surface cellulaire pour des fibroblastes actifs chez les souris ; et (vii) des facteurs de croissance.

4. Utilisation selon l'une quelconque des revendications précédentes, pour le traitement d'une maladie sélectionnée dans le groupe consistant en des allergies (rhinite allergique saisonnière (rhume des foins), conjonctivite allergique, rhinite vasomotrice et allergie alimentaire), l'éosinophilie, l'asthme, la polyarthrite rhumatoïde, le lupus érythémateux systémique, le lupus érythémateux discoïde, la colite ulcéreuse, la maladie de Crohn, les hémorroïdes, le prurit, la glomérulonéphrite, l'hépatite, la pancréatite, la gastrite, la vasculite, la myocardite, le psoriasis, l'eczéma, la fibrose induite par les radiations, la fibrose pulmonaire et d'autres troubles fibrotiques.

5. Utilisation selon l'une quelconque des revendications précédentes, dans laquelle l'agent comprend un premier domaine qui clive une protéine sélectionnée parmi SNAP-25, la synaptobrévine et la syntaxine.

6. Utilisation selon l'une quelconque des revendications précédentes, dans laquelle le deuxième domaine comprend une région H_{N} d'un polypeptide clostridial ou d'un fragment de celui-ci qui entraîne une translocation de l'activité d'inhibition de l'exocytose du premier domaine dans la cellule.

7. Utilisation selon l'une quelconque des revendications précédentes, pour l'inhibition d'une libération constitutive et régulée de cellules inflammatoires non neuronales.

8. Agent pour inhiber une sécrétion d'une cellule inflammatoire non neuronale, comprenant au moins un premier, un deuxième et un troisième domaine
où le premier domaine comprend une chaîne légère de neurotoxine clostridiale ou un fragment de celle-ci qui clive une ou plusieurs protéines essentielles à l'exocytose,
le deuxième domaine comprend un domaine de translocation sélectionné dans le groupe consistant en une partie H_{N} de chaîne lourde de toxine clostridiale, un domaine de translocation de toxine diphtérique, un domaine de translocation de l'exotoxine A de Pseudomonas, un domaine de translocation fusogénique de l'hémagglutinine du virus de la grippe, un domaine de translocation de peptide amphiphile, et des fragments de ceux-ci qui entraînent la translocation du premier domaine dans la cellule
et le troisième domaine comprend un fragment de ciblage qui se lie avec une haute spécificité et/ou affinité à un site de liaison sur la surface d'une cellule inflammatoire non neuronale, où le fragment de ciblage est sélectionné dans le groupe consistant en des anticorps, des anticorps monoclonaux, des fragments d'anticorps, des lectines, des hormones, des cytokines, des facteurs de croissance, des peptides, des glucides, des lipides, des glycones, et des composants du complément.

9. Agent selon la revendication 8, dans lequel le troisième domaine est tel que défini dans la revendication 3.

10. Composition pharmaceutique comprenant un agent selon la revendication 8 ou la revendication 9, en combinaison avec un véhicule pharmaceutiquement acceptable.

11. Utilisation d'un agent selon la revendication 8 ou la revendication 9 ou d'une composition pharmaceutique selon la revendication 10, pour la fabrication d'un médicament pour le traitement d'une maladie provoquée, exacerbée ou maintenue par une sécrétion à partir d'une cellule inflammatoire non neuronale.

12. Produit de recombinaison d'acide nucléique codant pour un agent selon la revendication 8 ou la revendication 9, ledit produit de recombinaison comprenant des séquences d'acide nucléique codant pour le premier, le deuxième et le troisième domaines.

13. Produit de recombinaison d'acide nucléique selon la revendication 12, en liaison fonctionnelle avec des séquences promotrices et de terminaison, et facultativement des séquences régulatrices, lesdites séquences promotrices, de terminaison et régulatrices étant fonctionnelles dans une cellule cible inflammatoire non neuronale afin d'entraîner l'expression dudit agent dans ladite cellule.

14. Agent destiné à être utilisé en thérapie génique, comprenant :
(i) une séquence d'acide nucléique codant pour un premier domaine comprenant une chaîne légère de neurotoxine clostridiale ou un fragment de celle-ci qui clive une ou plusieurs protéines essentielles à l'exocytose ;
(ii) un deuxième domaine associé à la séquence d'acide nucléique, où ledit deuxième domaine comprend un domaine de translocation sélectionné dans le groupe consistant en une partie H_{N} de chaîne lourde de toxine clostridiale, un domaine de translocation de toxine diphtérique, un domaine de translocation de l'exotoxine A de Pseudomonas, un domaine de translocation fusogénique de l'hémagglutinine du virus de la grippe, un domaine de translocation de peptide amphiphile, et des fragments de ceux-ci qui, après administration de l'agent à un patient, entraînent une translocation de la séquence d'acide nucléique dans une cellule cible inflammatoire non neuronale où, lorsqu'elle est présente dans ladite cellule cible inflammatoire non neuronale, l'expression de la séquence d'acide nucléique est obtenue dans celle-ci ; et
(iii) un troisième domaine qui comprend un fragment de ciblage qui se lie avec une haute spécificité et/ou affinité à un site de liaison sur la surface afin de cibler l'agent vers ladite cellule inflammatoire non neuronale, où le fragment de ciblage est sélectionné dans le groupe consistant en des anticorps, des anticorps monoclonaux, des fragments d'anticorps, des lectines, des hormones, des cytokines, des facteurs de croissance, des peptides, des glucides, des lipides, des glycones, et des composants du complément.

15. Agent selon la revendication 14, dans lequel la séquence d'acide nucléique est en liaison fonctionnelle avec des séquences promotrices et de terminaison, et facultativement des séquences régulatrices, lesdites séquences promotrices, de terminaison et régulatrices étant fonctionnelles dans la cellule cible inflammatoire non neuronale afin d'entraîner l'expression dudit agent dans ladite cellule cible inflammatoire non neuronale.

16. Agent selon la revendication 14 ou la revendication 15, dans lequel le troisième domaine est tel que défini dans la revendication 3.

17. Utilisation d'un agent selon l'une quelconque des revendications 14-16, pour la fabrication d'un médicament pour le traitement d'une maladie provoquée, exacerbée ou maintenue par une sécrétion à partir d'une cellule inflammatoire non neuronale.

18. Utilisation d'un produit de recombinaison d'acide nucléique selon la revendication 12 ou la revendication 13, pour la fabrication d'un médicament pour le traitement d'une maladie provoquée par, exacerbée ou maintenue par une sécrétion à partir d'une cellule inflammatoire non neuronale.
